(19) Europäisches Patentamt
European Patent Office
Office européen des brevets

(11) Veröffentlichungsnummer : **0 440 581 A1**

(12) # EUROPÄISCHE PATENTANMELDUNG

(21) Anmeldenummer : **91810050.4**

(22) Anmeldetag : **22.01.91**

(51) Int. Cl.$^5$ : **C12N 15/32**, C12N 15/62, A01N 63/00

Der Anmelder hat eine Erklärung nach Regel 28 (4) EPÜ (Herausgabe einer Probe nur an einen Sachverständigen) eingereicht.
Eingangsnummer(n) der Hinterlegung(en): DSM 3668, DSM 4571, DSM 4572, DSM 4573.

(30) Priorität : **31.01.90 CH 302/90**

(43) Veröffentlichungstag der Anmeldung : **07.08.91 Patentblatt 91/32**

(84) Benannte Vertragsstaaten : **AT BE CH DE DK ES FR GB GR IT LI LU NL SE**

(71) Anmelder : **CIBA-GEIGY AG**
**Klybeckstrasse 141**
**CH-4002 Basel (CH)**

(72) Erfinder : **Geiser, Martin, Dr.**
**Hauptstrasse 3A**
**CH-4107 Ettingen (CH)**
Erfinder : **Moser, Jacqueline**
**Laufenburgerstrasse 10/7**
**CH-4058 Basel (CH)**

(54) **Temperaturstabiles Bacillus thuringiensis-Toxin.**

(57) Die vorliegende Erfindung beschreibt ein mit Hilfe gentechnischer Verfahren hergestelltes, insektizides Toxin aus *Bacillus thuringiensis,* das im Gegensatz zu seinem natürlichen Gegenstück auch bei Temperaturen von > 25° C noch stabil ist sowie die das besagte Toxin kodierende DNA. Ein weiterer Gegenstand dieser Erfindung betrifft gentechnische Verfahren zur Herstellung eines solchen temperaturstabilen Toxins sowie die in diesem Verfahren eingesetzten Vektoren, Plasmide und Wirtsorganismen, welche die Toxin-kodierende DNA enthalten. Ebenfalls umfasst von der vorliegenden Erfindung sind insektizide Mittel, die besagtes temperaturstabiles *B.t.* Toxin als aktive Komponente enthalten sowie deren Verwendung in einem Verfahren zur Bekämpfung von Schadinsekten.

EP 0 440 581 A1

# TEMPERATURSTABILES BACILLUS THURINGIENSIS-TOXIN

Die vorliegende Erfindung betrifft ein mit Hilfe gentechnischer Verfahren hergestelltes, insektizides Toxin aus *Bacillus thuringiensis*, das im Gegensatz zu seinem natürlichen Gegenstück auch bei Temperaturen von > 25° C noch stabil ist sowie die das besagte Toxin kodierende DNA.

Ein weiterer Gegenstand dieser Erfindung betrifft gentechnische Verfahren zur Herstellung eines solchen temperaturstabilen Toxins sowie die in diesem Verfahren eingesetzten Vektoren, Plasmide und Wirtsorganismen, welche die Toxin-kodierende DNA enthalten.

Ebenfalls umfasst von der vorliegenden Erfindung sind insektizide Mittel, die besagtes temperaturstabiles *B.t.* Toxin als aktive Komponente enthalten sowie deren Verwendung in einem Verfahren zur Bekämpfung von Schadinsekten.

Die meisten der bisher bekannten *Bacillus thuringiensis* Stämme produzieren im Verlaufe ihrer Sporulation ein insektizides Toxin, das sog. δ-Endotoxin. Das insektizide Potential dieser Toxine wurde bereits sehr früh erkannt. Schon Ende der 20iger Jahre wurden *B. thuringiensis* Präparate als Bioinsektizide zur Bekämpfung verschiedener, durch Insekten verursachter Erkrankungen von Kulturpflanzen eingesetzt. Mittlerweile existieren auf diesem Indikationsgebiet bereits eine ganze Reihe von *B.t.* Präparaten, die aus Naturisolaten hergestellt werden.

Zu den am besten untersuchten *B. thuringiensis* Stämmen gehört zweifellos der *B.t.* Stamm HD1 subsp. *kurstaki*. Von diesem Stamm ist bekannt, dass er in seinem Genom bis zu drei homologe, Plasmid-kodierte Gene enthält, die insektizid wirksame Toxin-Proteine mit einem Molekulargewicht zwischen 130 und 134 Kilodalton kodieren. Diese Gene werden in der Literatur in drei Gruppen einklassifiziert und im allgemeinen Sprachgebrauch als 6.6 oder CryIA(c), 5.3 oder CryIA(b) und 4.5 oder CryIA(a) Gen bezeichnet, entsprechend der Grösse der nach HindIII Verdauung erhältlichen DNA Fragmente, auf welchen sich das 5'-Ende dieser Gene befindet. Es ist mittlerweile in allen drei Fällen gelungen die entsprechenden Gene zu isolieren, diese in *E.coli* zu klonieren und dort auch zu exprimieren [Adang *et al*, 1985 ; Kronstad JW and Whiteley, 1984 ; McLinden *et al*, 1985]. Es konnte dabei anhand der Wirkungsspektren gezeigt werden, dass die verschiedenen Toxinproteine sowohl in quantitativer als auch in qualitativer Hinsicht zum Teil sehr unterschiedliche Spezifitäten aufweisen.

Mit der Entwicklung neuer Transformationstechniken, die basierend auf einer Elektroporation nunmehr auch die Einklonierung von Genen in *B. thuringiensis* und/oder *B. cereus* erlauben [Schurter *et al*, 1989], ergab sich erstmals die Möglichkeit Toxingene aus *B. thuringiensis* direkt im natürlichen Wirt zu klonieren und dort auch zu exprimieren, was zahlreiche Vorteile mit sich bringt.

Im Verlaufe dieser Transformationsexperimente konnte ein interessantes Phänomen beobachtet werden. Kloniert man beispielsweise das CryIA(b)-Gen in einer Kristallkörper freien Mutanten von *B. thuringiensis* und bringt es dort bei einer normalen Kultivierungstemperatur zwischen 30° C und 33° C zur Expression, so lassen sich nach Lyse der *B.t.* Zellen nur sehr geringe Toxinproteinkonzentrationen im Lysat nachweisen. Werden die transformierten *B.t.* Zellen dagegen bei 25° C kultiviert, so lässt sich ohne weiteres ein normaler Proteinspiegel erreichen. Dieses Phänomen konnte bei den CryIA(a)- und CryIA(c)-Genen nicht beobachtet werden. Die von diesen Genen exprimierten Toxinproteine sind sowohl bei einer Kultivierungstemperatur von 25° C als auch bei Kultivierungstemperaturen von 30° C bis 33° C in vergleichbaren Konzentrationen im Lysat nachweisbar.

Untersuchungen haben gezeigt, dass die im Falle der CryIA(b)-Gene beobachtete Variabilität in der Proteinkonzentration nachweislich nicht mit der Gen-Transkription oder-Translation in Zusammenhang steht, die beobachteten Unregelmässigkeiten somit nicht auf der Expressionsebene erklärbar sind. Es liess sich daher nur vermuten, dass die Ursachen bei dem durch das CryIA(b)-Gen kodierten Proteinprodukt zu suchen sind, indem dies bei einer Temperatur von > 25° C offenbar gewisse Instabilitäten aufweist.

Es bestand somit die Aufgabe, das CryIA(b)-Toxingen in einer Weise zu modifizieren, dass von dem modifizierten Gen ein temperaturstabiles Toxinprotein kodiert und dies in normalem Umfang exprimiert wird. Diese Aufgabe konnte nunmehr im Rahmen der vorliegenden Erfindung überraschenderweise gelöst werden.

Die vorliegende Erfindung betrifft daher in erster Linie modifizierte CryIA(b)-Gene, die ein Genprodukt kodieren, welches bei einer Temperatur von > 25° C normale Stabilität aufweist und damit bei einer für *B. thuringiensis* Zellen bevorzugten Kultivierungstemperatur von 30° C bis 33° C in den üblichen Ausbeuten erhältlich ist, sowie Verfahren zur Herstellung besagter modifizierter CryIA(b)-Gene. Unter 'üblichen Ausbeuten' sollen im Rahmen dieser Erfindung Toxinkonzentrationen im *B.t.* Lysat verstanden werden, wie sie unter vergleichbaren Bedingungen auch bei der Expression der CryIA(a) bzw. CryIA(c)-Gene erreicht werden.

Ebenso umfasst sind Mutanten und Varianten besagter modifizierter CryIA(b) Gene, einschliesslich Teilsequenzen, die noch die charakteristischen Eigenschaften des Ausgangsgens aufweisen.

Ein weiterer Gegenstand dieser Erfindung umfasst rekombinante DNA Moleküle, die ein modifiziertes

CryIA(b)-Gen enthalten, das ein Genprodukt kodiert, welches bei einer Temperatur von > 25° C normale Stabilität aufweist und damit bei einer für *B. thuringiensis* Zellen bevorzugten Kultivierungstemperatur von 30° C bis 33° C in den üblichen Ausbeuten erhältlich ist.

Ebenso umfasst sind Vektoren und Plasmide, die besagtes modifiziertes CryIA(b)-Gen enthalten sowie die damit transformierten Wirtsorganismen.

Besonders bevorzugt im Rahmen dieser Erfindung sind *B. thuringiensis* oder *B. cereus*-Zellen, die mit einem rekombinanten DNA-Molekül transformiert sind, das ein CryIA(b)-Strukturgen enthält, welches ein modifiziertes, temperaturstabiles δ-Endotoxin-Polypeptid kodiert, das auch bei einer Kultivierungstemperatur von > 25° C, insbesondere aber bei einer für die Kultivierung der *B. thuringiensis* Zellen bevorzugten Temperatur von 30° C bis 33° C stabil und damit in den üblichen Ausbeuten erhältlich ist, oder aber ein Polypeptid, das diesem im wesentlichen homolog ist.

Die vorliegende Erfindung umfasst weiterhin die durch besagte modifizierte CryIA(b)-Gene kodierten temperaturstabilen Protoxinmoleküle, welche bei einer Temperatur von > 25° C normale Stabilität aufweisen und damit bei einer für *B. thuringiensis* Zellen bevorzugten Kultivierungstemperatur von 30° C bis 33° C in den üblichen Ausbeuten erhältlich sind, sowie Verfahren zur Herstellung dieser temperaturstabilen Protoxin-moleküle.

Ebenso umfasst sind Mutanten und Varianten besagter temperaturstabiler Protoxin-moleküle, einschliesslich Teilsequenzen, die noch die charakteristischen Eigenschaften des Ausgangsmaterials aufweisen.

Ein weiterer Gegenstand dieser Erfindung betrifft insektizide Mittel, die besagtes temperaturstabiles Protoxin enthalten sowie Verfahren zur Bekämpfung von Schadinsekten auf Kulturpflanzen unter Verwendung besagter insektizider Mittel.

**Kurze Beschreibung der Abbildungen und Sequenzprotokolle**

**Fig. 1** Konstruktionsschema von Plasmid pXI106.

für die Ligasereaktion verwendete DNA-Fragmente;

in das CryIA(b)-Gen eingebautes KpnI/HindIII Fragment aus Plasmid pXI54;

Pfeil gibt Leserichtung und Ausdehnung des CryIA(b)- bzw. des CryIA(c)-Gens an;

Δ bezeichnet die Lage der Deletion auf dem CryIA(b)-Gen

**SEQ ID NO : 1** KpnI/HindIII Fragment aus Plasmid pXI36. Dieses Fragment umfasst einen Bereich innerhalb des CryIA(b)- Gens aus *B. thuringiensis* subsp. *kurstaki* HD1, der sich von Position 2329 bis 2899 erstreckt und natürlicherweise eine Deletion von 78 Bp aufweist im Vergleich zu den anderen bekannten CryIA-Genen.

**SEQ ID NO : 2** KpnI/HindIII Fragment aus Plasmid pXI54. Dieses Fragment umfasst einen Bereich innerhalb des CryIA(c)- Gens aus *B. thuringiensis* subsp. *kurstaki* HD73 (vergl. Adang et al, 1985), der sich von Position 2564 bis 3212 erstreckt.

**SEQ ID NO : 3** zeigt die Unterschiede in der Aminosäuresequenz der durch die Plasmide pXI93 und pXI106 kodierten Genprodukte im Bereich 720 bis 916 [CryIA(b)-Toxin] bzw. 719 bis 942 [modifiziertes CryIA(b)-Toxin]

**SEQ ID NO : 4** zeigt die vollständige DNA Sequenz des HpaI/PstI Fragments aus Plasmid pXI106 nach Eliminierung der Deletion innerhalb der kodierenden Region des CryIA(b) Gens aus *B. thuringiensis* subsp. *kurstaki* HD1.

**SEQ ID NO : 5**

zeigt die Aminosäuresequenz des thermostabilen Genproduktes, welches durch das modifizierte CryIA(b)-Gen aus *B. thuringiensis* subsp. *kurstaki* HD1 kodiert wird.

Aus vergleichenden Untersuchungen der Aminosäuresequenzen der CryIA-Gene von Geiser *et al* (1986) sowie von Höfte und Whiteley (1989) ist bekannt, dass die Unterschiede zwischen diesen Genen im wesentlichen in der N-terminalen Region des Gens zu finden sind, während die C-terminale Hälfte in hohem Masse konservativ erscheint. Es wurde daher vermutet, dass der C-terminalen Region möglicherweise eine gewisse funktionale Signifikanz zukommen könnte.

Im Rahmen der vorliegenden Erfindung konnte jetzt aber überraschenderweise gezeigt werden, dass die C-terminale Region der CryIA-Gene offenbar direkt mit der Stabilität der kodierten Toxinproteine in Zusammenhang steht. Aufgrund dieser Erkenntnis ist es im Rahmen der vorliegenden Erfindung nunmehr gelungen, das CryIA(b)-Gen, welches ein temperaturlabiles Genprodukt kodiert, so zu modifizieren, dass die bei einer Kultivierungstemperatur von > 25° C beobachteten Instabilitäten des Toxin-Genproduktes eliminiert werden konnten.

Die vorliegende Erfindung betrifft somit in erster Linie ein neues, modifiziertes CryIA(b)-Gen, welches ein Genprodukt kodiert und exprimiert, das auch bei einer Kultivierungstemperatur von > 25° C, insbesondere aber bei einer für die Kultivierung der *B. thuringiensis* Zellen bevorzugten Temperatur von 30° C bis 33° C stabil und damit in den üblichen Ausbeuten erhältlich ist.

Aus der Literatur (Geiser *et al*, 1986) ist bekannt, dass der oben angesprochene Konservativismus der C-terminalen Region im CryIA(b)-Gen insofern durchbrochen ist, als das CryIA(b)-Gen im Vergleich zu den beiden anderen CryIA-Genen im C-Terminus eine kurze, 78 Basenpaare umfassende Deletion aufweist. Es hat sich nun im Rahmen dieser Erfindung überraschenderweise gezeigt, dass sich der beobachtete Temperaturdefekt des CryIA(b)-Genproduktes beheben lässt, indem man diese Deletion, -vorzugsweise mit Hilfe gentechnischer Methoden-, beseitigt. Dies kann z.B. durch Austausch eines entsprechenden Fragments aus dem CryIA(b)-Gen, welche diese Deletion aufweist, mit einem vollständig oder teilweise überlappenden Fragment aus einem CryIA(a)- oder einem CryIA(c)-Gen ohne Deletion erfolgen. Man erhält auf diese Weise ein neues, modifiziertes Gen, welches im Gegensatz zum bekannten CryIA(b)-Gen ein Toxin-Protein kodiert, das bei einer Temperatur von > 25° C normale Stabilität aufweist und damit bei einer für *B. thuringiensis* Zellen bevorzugten Kultivierungstemperatur von 30° C bis 33° C in den üblichen Ausbeuten erhältlich ist.

Unter einer 'normalen' Stabilität soll in diesem Fall eine Stabilität verstanden werden, die mit derjenigen der CryIA(a) bzw. CryIA(c)-Genprodukte vergleichbar ist.

Die vorliegende Erfindung betrifft somit insbesondere ein neues und modifiziertes CryIA(b)-Gen, welches dadurch gekennzeichnet ist, dass die im C-Terminus vorhandene Deletion durch Austausch eines entsprechenden Fragments aus dem CryIA(b)-Gen, welches diese Deletion aufweist, mit einem vollständig oder teilweise überlappenden Fragment aus einem CryIA(a)- oder einem CryIA(c)-Gen ohne Deletion oder mit einem diesen CryIA-Fragmenten homologen DNA-Fragment, beseitigt ist.

Unter homologen DNA-Fragmenten sollen im Rahmen dieser Erfindung Fragmente verstanden werden, die im wesentlichen eine Homologie von 60%, vorzugsweise aber eine Homologie von 80% und insbesondere eine Homologie von 90% aufweisen.

Ebenso umfasst von dieser Erfindung ist das durch besagtes modifiziertes CryIA(b)-Gen kodierte Protoxin, das bei einer Temperatur von > 25° C normale Stabilität aufweist und damit bei einer für *B. thuringiensis* Zellen bevorzugten Kultivierungstemperatur von 30° C bis 33° C in den üblichen Ausbeuten erhältlich ist

Neben dem Austausch ganz oder teilweise überlappender DNA-Fragmente aus dem C-Terminus der CryIA-Gene sind noch andere Lösungen zur Beseitigung der im CryIA(b)-Gen vorliegenden Deletion denkbar, wie z.B. ein Austausch der gesamte C-terminalen Region des CryIA(b) Gens.

Bevorzugt im Rahmen dieser Erfindung ist der Austausch eines Fragments aus dem CyrIA(b)-Gen, welches diese Deletion aufweist, mit einem vollständig oder teilweise überlappenden Fragment aus einem CryIA(c)-Gen ohne Deletion oder mit einem diesem Fragment homologen DNA-Fragment. Vorzugsweise handelt es sich dabei um ein Fragment, insbesondere aber um ein KpnI-HindIII Fragment, welches einen DNA-Abschnitt umfasst, der sich von Position 2769 bis 2846 auf dem CryIA(c)-Gen (Adang *et al*, 1985) erstreckt. Die Grösse des Fragments ist dabei nicht limitierend, da mittlerweile Verfahren existieren, welche die Herstellung von Fragmenten beliebiger Grösse, unabhängig von vorhandenen Restriktionsstellen, ermöglichen, wie z.B. der Polymerase Chain Reaction (PCR) Prozess.

In einer besonderen Ausführungsform der vorliegenden Erfindung wird die im CryIA(b)-Gen vorliegende Deletion durch den Austausch eines KpnI-HindIII Fragments aus dem in EP-A 342 633 publizierten CryIA(b)-Gen (Position 2329 bis 2899), das die in SEQ ID NO : 1 wiedergegebene DNA Sequenz einschliesslich der oben erwähnten Deletion aufweist, mit dem korrespondierenden KpnI-HindIII Fragment aus dem bei Adang *et al* (1985) publizierten CryIA(c) Gen (Position 2564 bis 3212), dessen DNA Sequenz in SEQ ID NO : 2 gezeigt ist, eliminiert.

Das auf diesem Wege entstandene neue und modifizierte CryIA(b)-Gen, dessen vollständige DNA-Sequenz in SEQ ID NO : 4 wiedergegeben ist, kodiert ein Protoxinmolekül, das im Vergleich zum Wildtyp-Genprodukt die in SEQ ID NO : 3 gezeigten Unterschiede in der Aminosäuresequenz aufweist und das auch bei einer Temperatur von > 25° C, insbesondere aber bei einer für die Kultivierung von *B.thuringiensis* Zellen bevorzugten Temperatur zwischen 30° C und 33° C normale Stabilität aufweist.

Die Aminosäuresequenz eines im Rahmen dieser Erfindung bevorzugten CryIA(b) Genproduktes ist in ID SEQ NO : 5 wiedergegeben.

Der Austausch eines Fragments auf dem CyrIA(b)-Gen, welches gegenüber den korrespondierenden CryIA(a)- und CryIA(c)-Genen eine Deletion von 78 Basenpaaren aufweist, mit einem vollständig oder teilweise überlappenden Fragment aus der entsprechenden Region eines CryIA(a)- oder CryIA(c)-Gens, kann vorzugsweise unter Anwendung gentechnischer Verfahren durchgeführt werden.

So kann beispielsweise der Austausch eines KpnI-HindIII Fragments aus dem in EP-A 342 633 publizierten CryIA(b)-Gen (Position 2329 bis 2899), das die oben erwähnte Deletion aufweist, mit dem korrespondierenden KpnI-HindIII Fragment aus dem bei Adang *et al* (1985) publizierten CryIA(c) Gen (Position 2564 bis 3212), der im Rahmen der vorliegenden Erfindung bevorzugt ist, dadurch erreicht werden, dass man geeignete Fragmente unterschiedlicher Grösse aus den besagten Genen isoliert und diese anschliessend in einer dem Fachmann bekannten Verknüpfungsreaktion wieder so miteinander verbindet, dass ein vollständiges und funktionsfähiges Toxingen resultiert.

In einer bevorzugten Ausführungsform der vorliegenden Erfindung werden dabei die folgenden Fragmente in einer Ligasereaktion miteinander verknüpft :

(a) das nach Restriktionsverdauung erhältliche, grosse PstI-KpnI Fragment aus dem Plasmid pXI93 [Schurter *et al*, 1989] das im wesentlichen den N-terminalen Teil des CryIA(b)-Gens umfasst, nicht jedoch die oben erwähnte Deletion ;

(b) ein 648 Bp umfassendes KpnI-HindIII Fragment aus dem CryIA(c)-Gen, welches eine DNA-Region innerhalb des C-Terminus abdeckt, die der homologen Region auf dem CryIA(b)-Gen weitgehend homolog ist, mit Ausnahme der dort befindlichen Deletion ;

(c) ein 1460 Bp umfassendes HindIII-PstI Fragment aus dem Plasmid pXI36 [EP-A 238 441], welches einen Grossteil des C-Terminus des CryIA(b)-Gens enthält, nicht jedoch die Region mit der Deletion.

Nach Verknüpfen dieser Fragmente in einer Ligasereaktion und Einklonierung in einen geeigneten Wirt wie z.B. einen geeigneten *E.coli*-Stamm erhält man ein vollständiges und modifiziertes CryIA(b)-Gen, das im Gegensatz zum Wildtyp-Gen keine Deletion im C-Terminus aufweist und dessen Genprodukt dementsprechend bei einer Temperatur von > 25° C, insbesondere aber bei einer für die Kultivierung von *B.thuringiensis* Zellen bevorzugten Temperatur zwischen 30° C und 33° C, stabil ist.

Für die Transformation und Expression des modifizierten CryIA(b)-Gens in *B. thuringiensis* ist es vorteilhaft, wenn mindestens eines der oben erwähnten Fragmente bereits DNA Sequenzen enthält, die für eine Replikation und Expression des modifizierten Toxingens in *B. thuringiensis* geeignet sind. Besagte Sequenzabschnitte befinden sich beispielsweise auf dem unter Punkt (a) genannten grossen PstI-KpnI Fragment aus dem Plasmid pXI93, einem bifunktionellen Plasmid, dessen Zusammensetzung und Konstruktion bei Schurter *et al* (1989) sowie in EP-A 342 633 beschrieben ist.

Steht das für die Herstellung eines chimären Gens benötigte Ausgangsmaterial nicht in einer Menge zur Verfügung, die für die beabsichtigte genetische Manipulation ausreicht, kann dieses vorteilhafterweise zunächst in einen geeigneten Vektor eingebaut und der Vektor anschliessend durch Replikation in einer heterologen Wirtszelle amplifiziert werden. Am besten geeignet für die Amplifikation von Genen oder Genfragmenten sind Bakterien- oder Hefezellen. Wenn eine ausreichende Menge der benötigten Gene oder Genfragmente zur Verfügung steht, können diese für die Konstruktion des chimären Gens verwendet werden.

Alternativ dazu können die benötigten Genfragmente beispielsweise auch mit Hilfe einer 'Polymerase Chain Reaction' (PCR) erhalten werden. Dabei handelt es sich um ein Verfahren, mit dessen Hilfe eine bis zu $10^5$-fache Amplifikation von DNA-Fragmenten erreicht werden kann. Der Polymerase Chain Reaction Prozess ist im US-Patent 4,683,202 im Detail beschrieben. Gebrauchsfertige Kits zur Durchführung des PCR-Verfahrens sind im Handel erhältlich [Gene Amp™ DNA Amplification Reagent Kit, Perkin Elmer Cetus, Norwalk].

Bevor ein chimäres Gen in *B. thuringiensis* bzw. *B. cereus* Zellen oder einen anderen geeigneten Wirtsorganismus transformiert werden kann, wird es vorzugsweise in einen geeigneten Vektor integriert, der gegebenenfalls mit dem für die Amplifikation verwendeten Vektor identisch sein kann. Dabei kann entweder bereits der Zusammenbau des chimären Gens im Vektormolekül erfolgen oder aber das bereits vollständig zusammengebaute, chimäre Gen wird als Einheit in den Vektor integriert. Besonders bevorzugt ist ein Einbau chimärer Gene in bifunktionelle Vektoren.

Einige Beispiele für bakterielle Wirtszellen, die für eine Klonierung von Genen oder Genfragmenten geeignet sind, umfassen Bakterien, ausgewählt aus den Gattungen *Escherichia*, wie *E. coli, Agrobacterium,* wie *A. tumefaciens* oder *A. rhizogenes, Pseudomonas*, wie *Pseudomonas spp., Bacillus,* wie *B. megaterium* oder *B. subtilis*, etc. sowie Hefezellen, z.B. aus der Gattung *Saccharomyces* (EP-A 0 238 441). Darüberhinaus können *B. thuringiensis* bzw. *B. cereus* selbst als Wirtszellen verwendet werden. Verfahren der Klonierung heterologer Gene in Bakterien sind in den US Patenten 4,237,224 und 4,468,464 beschrieben.

Die Replikation von Genen in *E. coli*, die das kristalline Protein von *B. thuringiensis* kodieren, wird von Wong *et al.* (1983) beschrieben.

Für die Klonierung von Genen oder Genfragmenten kann jeder beliebige Vektor verwendet werden, sofern er geeignete Restriktionsschnittstellen aufweist und in der Lage ist in einer geeigneten Wirtszelle, wie z.B. einem Bakterium oder einer Hefezelle zu replizieren. Der Vektor kann beispielsweise von einem Phagen oder einem Plasmid abgeleitet sein. Beispiele für Vektoren, die von Phagen abgeleitet und im Rahmen dieser Erfindung verwendet werden können, sind Vektoren, die von M13- und von λ-Phagen abgeleitet sind- Einige geeignete Vektoren, die von M13-Phagen abgeleitet sind, schliessen M13mp18 und M13mp19 ein. Einige von λ-Phagen abgeleitete geeignete Vektoren schliessen λgt11, λgt7 und λCharon4 ein.

Von den Vektoren, die von Plasmiden abgeleitet und ganz besonders für die Replikation in Bakterien geeignet sind, seien hier beispielhaft pBR322 (Bolivar *et al*, 1977), pUC18 und pUC19 (Norrander *et al*, 1983) sowie Ti-Plasmide (Bevan *et al*, 1983) genannt, ohne dadurch jedoch den Erfindungsgegenstand in irgendeiner Weise zu limitieren. Die bevorzugten Vektoren zur Amplifikation von Genen in Bakterien sind pBR322, pUC18 und pUC19.

Für eine Klonierung direkt in *B. thuringiensis* und/oder *B. cereus* sind in erster Linie Direktklonierungsvektoren zu nennen, wie z.B. pBD347, pBD348, pBD64 sowie pUB1664, sowie insbesondere 'shuttle'-Vektoren, die beispielsweise bei Schurter *et al* (1989) sowie in EP-A 342 633 im Detail beschrieben sind, ohne jedoch darauf beschränkt zu sein.

Besonders bevorzugt im Rahmen dieser Erfindung sind die bifunktionellen ('shuttle') Vektoren pXI61 und pXI93 [Schurter *et al* (1989)], die, transformiert in *B. thuringiensis* var. kurstaki HD1 cryB bzw. *B. cereus* 569 K, bei der als Internationale Hinterlegungsstelle anerkannten 'Deutschen Sammlung von Mikroorganismen' (Braunschweig, BRD) gemäss den Bestimmungen des Budapester Vertrages unter der Nummer DSM 4573 (pXI61, transformiert in *B. thuringiensis* var. kurstaki HD1 cryB) bzw. DSM 4571 (pXI93, transformiert in *B. thuringiensis* var. kurstaki HD1 cryB) und DSM 4573 (pXI93, transformiert in *B. cereus* 569 K) hinterlegt sind.

Um ein für die Replikation in Bakterien geeignetes chimäres Gen zu konstruieren, werden eine Promotorsequenz, eine 5'-nichttranslatierte Sequenz, eine kodierende Sequenz und eine 3'-nichttranslatierte Sequenz in einen Vektor eingefügt oder in der richtigen Reihenfolge in einem der zuvor beschriebenen Vektoren zusammengebaut. Geeignete Vektoren sind erfindungsgemäss solche, die in der Lage sind, sich in der Wirtszelle zu replizieren.

Der Promotor, die 5'-nichttranslatierte Region, die kodierende Region und die 3'-nichttranslatierte Region, können gegebenenfalls zuerst ausserhalb des Vektors in einer Einheit zusammengefasst und dann in den Vektor eingefügt werden. Alternativ dazu können aber auch Teile des chimären Gens einzeln in den Vektor eingefügt werden.

Im Falle der *B. thuringiensis* bzw. *B. cereus* Klonierungsvektoren kann dieser Verfahrensschritt entfallen, da die gesamte aus *B. thuringiensis* isolierte Einheit, bestehend aus einer 5'-nicht-translatierten Region, der kodierenden Region und einer 3'-nicht-translatierten Region, in den Vektor eingespleisst werden kann.

Der Vektor enthält darüberhinaus vorzugsweise auch ein Markergen, welches der Wirtszelle eine Eigenschaft verleiht, durch die man die mit dem Vektor transformierten Zellen erkennen kann. Bevorzugt sind Markergene, die eine Antibiotikaresistenz kodieren. Einige Beispiele für geeignete Antibiotika sind Ampicillin, Chloramphenicol, Erythromycin, Tetrazyklin, Hygromycin, G418 und Kanamycin.

Ebenfalls bevorzugt sind Markergerie, die Enzyme mit einem chromogenen Substrat, wie z.B. X-gal (5-Brom-4-chlor-3-indolyl-ß-D-galaktosid), kodieren. Die transformierten Kolonien können dann sehr einfach anhand einer spezifischen Farbreaktion nachgewiesen werden.

Die Einfügung oder der Zusammenbau des Gens im Vektor wird mit Hilfe von Standardverfahren durchgeführt, beispielsweise durch die Verwendung von rekombinanter DNA (Maniatis *et al*, 1982) in Verbindung mit der homologen Rekombination (Hinnen *et al*, 1978).

Die Verfahren der rekombinanten DNA-Technologie beruhen darauf, dass der Vektor zunächst geschnitten und die gewünschte DNA-Sequenz zwischen die geschnittenen Stücke des Vektors eingefügt wird ; die Enden der gewünschten DNA-Sequenz werden anschliessend mit den entsprechenden Enden des Vektors verknüpft.

Der Vektor wird vorzugsweise mit geeigneten Restriktionsendonukleasen geschnitten. Geeignete Restriktionsendonukleasen sind beispielsweise solche, die glatte Enden bilden, wie SmaI, HpaI und EcoRV, sowie solche, die kohäsive Enden bilden, wie EcoRI, SacI und BamHI.

Die gewünschte DNA-Sequenz existiert normalerweise als Teil eines grösseren DNA-Moleküls, wie eines Chromosoms, eines Plasmids, eines Transposons oder eines Phagen. Die gewünschte DNA-Sequenz wird in diesen Fällen aus ihrer ursprünglichen Quelle herausgeschnitten und gegebenenfalls so modifiziert, dass ihre Enden mit denen des geschnittenen Vektors verbunden werden können. Wenn die Enden der gewünschten DNA-Sequenz und des geschnittenen Vektors glatte Enden sind, können sie beispielsweise mit für glatte Enden spezifischen Ligasen, wie der T4 DNA-Ligase, miteinander verbunden werden.

Die Enden der gewünschten DNA-Sequenz können auch in der Form von kohäsiven Enden mit den Enden des geschnittenen Vektors verbunden werden, in welchem Fall eine für kohäsive Enden spezifische Ligase, die auch T4 DNA-Ligase sein kann, benutzt wird. Eine andere geeignete, für kohäsive Enden spezifische Ligase ist beispielsweise die *E. coli* DNA-Ligase.

Kohäsive Enden werden zweckmässigerweise gebildet, indem die gewünschte DNA-Sequenz und der Vektor mit der gleichen Restriktionsendonuklease geschnitten werden. In diesem Fall haben die gewünschte DNA-Sequenz und der geschnittene Vektor kohäsive Enden, die einander komplementär sind.

Die kohäsiven Enden können auch konstruiert werden, indem mit Hilfe der terminalen Desoxynukleotidyl-Transferase komplementäre, homopolymere Schwänze an die Enden der gewünschten DNA-Sequenz und des geschnittenen Vektors angehängt werden. Alternativ können auch kohäsive Enden hergestellt werden, indem eine synthetische Oligonukleotid-Sequenz, die von einer bestimmten Restriktionsendonuklease erkannt wird und die unter der Bezeichnung Linker bekannt ist, angehängt und die Sequenz mit der Endonuklease gespalten wird (siehe beispielsweise Maniatis et al., 1982).

Liegt der auf die zuvor beschriebene Weise hergestellte Vektor enthaltend ein modifiziertes CryIA(b)-Gen in ausreichender Menge vor, so kann er für die Transformation von *Bacillus thuringiensis* oder *B. cereus* Zellen verwendet werden. Diese wird vorzugsweise mit Hilfe einer Elektroportation durchgeführt, wie sie z.B. bei Schurter *et al*, 1989 beschrieben ist.

In einer spezifischen und im Rahmen dieser Erfindung bevorzugten Ausführungsform werden die *B. thuringiensis*-Zellen zunächst in einem geeigneten Nährmedium bei ausreichender Belüftung und bei einer geeigneten Temperatur, vorzugsweise von 20°C bis 35°C, inkubiert, bis eine optische Dichte ($OD_{550}$) von 0.1 bis 1.0 erreicht ist. Das Alter der für die Elektroporation vorgesehenen *Bacillus*-Kulturen hat einen deutlichen Einfluss auf die Transformationsfrequenz. Besonders bevorzugt ist daher eine optische Dichte der *Bacillus*-Kulturen von 0.1 bis 0.3, insbesondere aber von 0.2. Es sei jedoch darauf hingewiesen, dass sich auch mit *Bacillus*-Kulturen aus anderen Wachstumsphasen, insbesondere auch mit Uebernachtkulturen gute Transformationsfrequenzen erzielen lassen.

Als Ausgangsmaterial verwendet man in der Regel frische Zellen oder Sporen, es kann aber ebensogut auch auf tiefgefrorenes Zellmaterial zurückgegriffen werden. Dabei handelt es sich vorzugsweise um Zellsuspensionen von *B. thuringiensis* und/oder *B. cereus* Zellen in geeigneten Flüssigmedien, welchen vorteilhafterweise ein bestimmter Anteil eines 'Frostschutzmittels' zugesetzt wird.

Geignete Frostschutzmittel sind in erster Linie Gemische von osmotisch aktiven Komponenten und DMSO in Wasser oder einer geeigneten Pufferlösung. Weitere geeignete Komponenten, die für eine Verwendung in Frostschutzmittellösungen in Frage kommen, umfassen Zucker, mehrwertige Alkohole,wie z.B. Glycerin, Zuckeralkohole, Aminosäuren und Polymere, wie z.B. Polyethylenglykol.

Geht man von *B. thuringiensis*-Sporen aus, so werden diese zunächst in einem geeigneten Medium inokuliert und über Nacht bei einer geeigneten Temperatur, vorzugsweise von 25°C bis 28°C und ausreichender Belüftung inkubiert. Dieser Ansatz wird anschliessend verdünnt und in der oben beschriebenen Weise weiterbehandelt.

Zur Induktion der Sporulation bei *B. thuringierisis* können alle Medien verwendet werden, die eine solche Sporulation hervorrufen. Bevorzugt im Rahmen dieser Erfindung ist ein GYS-Medium nach Yousten AA und Rogoff MH, (1969).

Der Sauerstoffeintrag in das Kultivierungsmedium erfolgt in der Regel durch Bewegen der Kulturen, z.B. auf einer Schüttelmaschine, wobei Umdrehungsgeschwindigkeiten zwischen 50 Upm und 300 Upm bevorzugt sind.

Die Kultivierung von *B. thuringiensis*-Sporen sowie vegetativer Mikroorganismenzellen im Rahmen der vorliegenden Erfindung erfolgt nach bekannten, allgemein gebräuchlichen Methoden, wobei aus Gründen der Praktikabilität vorzugsweise flüssige Nährmedien verwendet werden.

Die Zusammensetzung der Nährmedien kann je nach verwendetem *B. thuringiensis*- bzw. *B. cereus* Stamm leicht variieren. Allgemein werden komplexe Medien mit wenig definierten, leicht assimilierbaren Kohlenstoff-(C-) und Stickstoff-(N-) Quellen bevorzugt, wie sie üblicherweise für die Kultivierung aerober *Bacillus*-Arten eingesetzt werden.

Ausser dem im Rahmen der vorliegenden Erfindungen bevorzugt verwendeten LB-Medium können auch alle anderen, für die Kultivierung von *B. thuringiensis* und/oder *B. cereus* geeigneten Kulturmedien verwendet werden, wie z.B. Antibiotic Medium 3, SCGY-Medium u.a. Die Aufbewahrung sporulierter *B. thuringiensis*-Kulturen erfolgt bevorzugterweise auf GYS Medien (Schrägagar) bei einer Temperatur von 4°C. [Die genaue Zusammensetzung der genannten Medien ist im Abschnitt "Medien und Pufferlösungen" wiedergegeben]

Nachdem die Zellkultur die gewünschte Zelldichte erreicht hat, werden die Zellen mit Hilfe einer Zentrifugation geerntet und in einer geeigneten Pufferlösung suspendiert, die zuvor vorzugsweise mit Eis gekühlt wird-Besonders geeignete Pufferlösungen im Rahmen dieser Erfindung sind osmotisch stabilisierte Phosphatpuffer, die als stabilisierendes Agens Zucker, wie z.B. Glucose oder Saccharose oder Zuckeralkohole, wie z.B. Mannit enthalten und auf pH-Werte von 5.0 bis 8.0 eingestellt sind. Ganz besonders bevorzugt sind Phosphatpuffer vom PBS-Typ mit einem pH-Wert von 5.0 bis 8.0, vorzugsweise von pH 5.5 bis 6.5, die Saccharose als stabilisierendes Agens in einer Konzentration von 0.1 M bis 1.0 M, vorzugsweise aber von 0.3 M bis 0.5 M enthalten.

Die Inkubationsdauer der *Bacillus*-Zellen vor und nach der Elektroporation beträgt vorzugsweise 0.1 bis 30 Minuten, insbesondere aber 10 Minuten. Die Temperatur ist in einem weiten Bereich frei wählbar. Bevorzugt ist ein Temperaturbereich von 0°C bis 35°C, vorzugsweise von 2°C bis 15°C und ganz besonders bevorzugt von 4°C.

Aliquots der suspendierten Bacillus-Zellen werden anschliessend in Küvetten oder beliebige andere, geeignete Gefässe überführt und mit der zu transformierenden DNA für einen geeigneten Zeitraum, vorzugsweise für einen Zeitraum von 0.1 bis 30 Minuten, insbesondere aber von 5 bis 15 Minuten, und bei einer geeigneten Temperatur, vorzugsweise bei einer Temperatur von 0°C bis 35°C, insbesondere aber bei einer Temperatur von 2°C bis 15°C und ganz besonders bevorzugt bei einer Temperatur von 4°C, gemeinsam inkubiert.

Beim Arbeiten mit tiefen Temperaturen ist es vorteilhaft bereits vorgekühlte Küvetten oder beliebige andere, geeignete und vorgekühlte Gefässe zu verwenden.

Die für eine *B. thuringiensis* oder *B. cereus* Transformation bevorzugte DNA-Konzentration liegt in einem Bereich zwischen 1 ng und 20 µg. Besonders bevorzugt ist eine DNA-Konzentration von 10 ng bis 2 µg.

Danach wird der gesamte Ansatz enthaltend *B. thuringiensis*- und/oder *B. cereus*-Zellen sowie die zu transformierende Plasmid-DNA in eine Elektroporationsapparatur eingebracht und einer Elektroporation unterzogen, d.h. kurzzeitig einem elektrischen Impuls ausgesetzt.

Elektroporationsapparaturen, die für eine Verwendung in dem erfindungsgemässen Verfahren geeignet sind, werden mittlerweile bereits von verschiedenen Herstellern angeboten, wie z.B. der Fa. Bio Rad (Richmond, CA, USA ; 'Gene Pulser Apparatus'), Biotechnologies and Experimental Research, Inc. (San Diego, CA, USA ; 'BTX Transfector 100'), Promega (Madison, WI, USA ;'X-Cell 2000 Electroporation System'), etc..

Selbstverständlich kann in dem erfindungsgemässen Verfahren auch jedes beliebige andere geeignete Gerät verwendet werden.

Die Kapazitätseinstellung am Kondensator beträgt vorteilhafterweise 1 µF bis 250 µF, insbesondere aber 1 µF bis 50 µF und ganz besonders bevorzugt 25 µF. Die Wahl der Ausgangsspannung ist in weiten Bereichen frei wählbar. Bevorzugt ist eine Ausgangsspannung $V_0$ von 0.2 kV bis 50 kV, insbesondere aber von 0.2 kV bis 2.5 kV und ganz besonders bevorzugt von 1.2 kV bis 1.8 kV. Der Abstand der Elektrodenplatten hängt u.a. ab von der Dimensionierung der Elektroporationsapparatur. Er beträgt vorteilhafterweise zwischen 0.1 cm und 1.0 cm, vorzugsweise zwischen 0.2 cm und 1.0 cm. Besonders bevorzugt ist ein Plattenabstand von 0.4 cm. Aus dem Abstand der Elektrodenplatten und der am Kondensator eingestellten Ausgangsspannung ergeben sich die Feldstärkewerte, die auf die Zellsuspension einwirken. Diese liegen vorteilhafterweise in einem Bereich zwischen 100 V/cm und 50'000 V/cm. Besonders bevorzugt sind Feldstärken von 100 V/cm bis 10'000 V/cm, insbesondere aber von 3'000 V/cm bis 4'500 V/cm.

Die im Rahmen des erfindungsgemässen Verfahrens bevorzugte exponentielle Abklingzeit liegt zwischen ca. 2 ms und ca. 50 ms, insbesondere aber zwischen ca. 8 ms und ca. 30 ms.

Ganz besonders bevorzugt ist eine exponentielle Abklingzeit von ca. 14 ms bis ca. 20 ms.

Die Feinabstimmung der frei wählbaren Parameter, wie z.B. Kapazität, Ausgangsspannung, Plattenabstand, etc. hängt bis zu einem gewissen Grad von der Architektur der verwendeten Geräte ab und kann daher von Fall zu Fall in bestimmten Grenzen variieren. Die angegebenen Grenzwerte können daher in gewissen Fällen auch über- oder unterschritten werden sofern dies zum Erreichen optimaler Feldstärkewerte erforderlich sein sollte.

Der eigentliche Elektroporationsvorgang kann ein- bis mehrmals wiederholt werden, bis eine für das jeweilige System optimale Transformationsfrequenz erreicht ist.

Im Anschluss an die Elektroporation kann vorteilhafterweise eine Nachinkubation der behandelten *Bacillus*-Zellen durchgeführt werden, vorzugsweise für einen Zeitraum von 0.1 bis 30 Minuten, bei einer Temperatur von 0°C bis 35°C, vorzugsweise von 2°C bis 15°C. Anschliessend werden die elektroporierten Zellen mit einem geeigneten Medium verdünnt und nochmals für einen geeigneten Zeitraum, vorzugsweise von 2 bis 3 Stunden unter ausreichender Belüftung und bei einer geeigneten Temperatur, vorzugsweise von 20°C bis 35 °C, inkubiert.

Nach der Elektroporation werden die behandelten *Bacillus thuringiensis* oder *B. cereus* Zellen auf ein selektives Sporulationsmedium übertragen und dort bis zur vollständigen Sporulation bei einer Temperatur von 10°C bis 40°C, vorzugsweise aber bei einer Temperatur von 20°C bis 35°C und ganz besonders bevorzugt bei einer Temperatur von 30°C bis 33°C inkubiert. Das Sporulationsmedium enthält als Selektivsubstanz vorzugsweise eine der oben genannten Antibiotika, abhängig vom verwendeten Vektor, sowie gegebenenfalls ein geeignetes Verfestigungsmittel, wie z.B. Agar, Agarose, Gelatine, etc.

Im Zuge der Sporulation kommt es zur Autolyse der sporulierenden Zellen, was für das nachfolgende Screening von grossem verfahrenstechnischen Vorteil ist, da ein künstliches Aufbrechen der Zellen entfällt. Bei Klonen, die das gesuchte Protoxingen enthalten und unter der Kontrolle ihres natürlichen Promotors exprimieren, liegen die gebildeten Kristallproteine frei zugänglich im Medium vor. Diese frei im Medium vorliegenden Kristallproteine können dann z.B. mit Hilfe von Membranfiltern oder durch andere geeignete Massnahmen, wie z.B. durch Abzentrifugieren aus der Kulturbrühe abgetrennt werden. Geeignete Membranfilter sind beispielsweise Nylon- oder Nitrozellulose-Membranen. Membranen dieser Art sind frei käuflich.

Die auf diese Weise isolierten Kristallproteine können dann sehr einfach im Rahmen eines geeigneten Immunassays identifiziert und quantifiziert werden.

Bevorzugt im Rahmen dieser Erfindung ist Immunassay auf der Grundlage eines ELISA Assays unter Verwendung Protoxin-spezifischer Antikörper, der z.B. in "Antibodies : A Laboratory Manual", eds. Harlow E and Lane D ; Cold Spring Harbor Laboratories, (1988) im Detail beschrieben ist. Bei den in diesem Immunassay verwendeten Antikörpern handelt es sich zum einen um spezifisch gegen *B.t.* Protoxin gerichtete monoklonale Antikörper sowie um einen mit einem spezifischen Marker versehenen anti-Immunglobulin Antikörper, vorzugsweise aber um einen Peroxidase-markierten anti-Maus IgG Antikörper aus Kaninchen, der z.B. bei der DIANAVA GmbH [Hamburg, BRD ; cat.# 315-035-045] erhältlich ist.

Besonders bevorzugt im Rahmen des erfindungsgemässen Verfahrens ist die Verwendung monoklonaler Antikörper, die ganz spezifisch einen bestimmten Teil des Proteinmoleküls erkennen. Diese Antikörper können entweder einzeln oder aber in Form eines Gesmisches verwendet werden. Darüberhinaus können aber selbstverständlich auch polyklonale Antiseren in dem Immunassay verwendet werden. Auch Gemische basierend auf monoklonalen und polyklonalen Antikörpern sind denkbar.

Verfahren zur Herstellung monoklonaler Antikörper gegen *Bacillus thuringiensis* Protoxin Proteine sind bekannt und z.B. bei Huber-Lukač *et al* (1986) im Detail beschrieben. Diese Verfahren lassen sich auch im vorliegenden Fall anwenden. Darüberhinaus können im Rahmen dieser Erfindung selbstverständlich auch andere geeignete Immunassays verwendet werden.

Bei Anwendung der zuvor beschriebenen Verfahrensmassnahmen erhält man ein modifiziertes *B.t.* Toxin, das auch bei einer Temperatur von > 25° C normale Stabilität aufweist und damit bei einer für *B. thuringiensis* Zellen bevorzugten Kultivierungstemperatur von 30° C bis 33° C in den üblichen Ausbeuten erhältlich ist.

Besonders bevorzugt im Rahmen dieser Erfindung ist ein modifiziertes *B.t.* Toxin, das die in SEQ ID NO: 5 wiedergegebene Aminosäuresequenz aufweist sowie Mutanten und Varianten davon, die strukturelle Veränderungen erfahren haben, welche die spezifischen und erfindungsgemässen Eigenschaften des Ausgangsmoleküls nicht wesentlich verändern.

*Bacillus thuringiensis* und *B. cereus*-Zellen, die mit Hilfe des zuvor beschriebenen Verfahrens transformiert worden sind, sowie die von diesen transformierten *Bacillus*-Zellen produzierten. Toxine eignen sich in hervorragender Weise für die Bekämpfung von Insekten, insbesondere aber zur Bekämpfung von Insekten aus den Ordnungen *Lepidoptera*.

Ein weiterer Gegenstand der vorliegenden Erfindung betrifft somit ein Verfahren zur Bekämpfung von Insekten, das sich dadurch kennzeichnet, dass man Insekten oder deren Lebensraum mit

a) *B. thuringiensis* oder *B. cereus*-Zellen oder mit einem Gemisch von beiden behandelt, die mit einem rekombinanten DNA-Molekül transformiert sind, das ein CryIA(b)-Strukturgen enthält, welches für ein modifiziertes, temperaturstabiles δ-Endotoxin-Polypeptid kodiert, das auch bei einer Kultivierungstemperatur von > 25° C, insbesondere aber bei einer für die Kultivierung der *B. thuringiensis* Zellen bevorzugten Temperatur von 30° C bis 33° C stabil und damit in den üblichen Ausbeuten erhältlich ist, oder aber ein Polypeptid, das diesem im wesentlichen homolog ist ; oder aber

b) mit zellfreien Kristalkörperpräparaten, die ein modifiziertes, temperaturstabiles Protoxin enthalten, das von besagten transformierten *Bacillus*-Zellen produziert wird.

Ebenso umfasst von der vorliegenden Erfindung sind insektizide Mittel, die neben den üblicherweise verwendeten Trägermitteln, Verteilungsmitteln oder Träger- und Verteilungsmitteln als aktive Komponente

a) *B. thuringiensis* oder *B. cereus*-Zellen oder ein Gemisch von beiden enthalten, die mit einem rekombinanten DNA-Molekül transformiert sind, das ein CryIA(b)-Strukturgen enthält, welches für ein modifiziertes, temperaturstabiles δ-Endotoxin-Polypeptid kodiert, das auch bei einer Kultivierungstemperatur von > 25° C, insbesondere aber bei einer für die Kultivierung der *B. thuringiensis* Zellen bevorzugten Temperatur von 30° C bis 33° C stabil und damit in den üblichen Ausbeuten erhältlich ist, oder aber ein Polypeptid, das diesem im wesentlichen homolog ist ; oder aber

b) zellfreie Kristallkörperpräparate, die ein modifiziertes, temperaturstabiles Protoxin enthalten, das von besagten transformierten *Bacillus*-Zellen produziert wird.

Für die Anwendung als Insektizide werden die transformierten Mikroorganismen, die das rekombinante *B. thuringiensis* Toxin-Gen enthalten, vorzugsweise transformierte lebende oder tote *B. thuringiensis* oder *B. cereus*-Zellen, einschliesslich Gemischen aus lebenden und toten *B. thuringiensis* und *B. cereus*-Zellen, sowie die von besagten transformierten Zellen produzierten Toxin-Proteine in unveränderter Form oder vorzugsweise zusammen mit den in der Formulierungstechnik üblichen Hilfsstoffen, eingesetzt und in an sich bekannter Weise formuliert, z.B. zu Suspensionskonzentraten, streichbaren Pasten, direkt versprühbaren oder verdünnbaren Lösungen, benetzbaren Pulvern, löslichen Pulvern, Stäubemitteln, Granulaten, und auch Verkapselungen in z.B. polymeren Stoffen.

Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt.

Es können darüberhinaus selbstverständlich auch insektizide Gemische verwendet werden, bestehend aus transformierten, lebenden oder toten *B. thuringiensis* und/oder *B. cereus* Zellen sowie aus zellfreien Kristallkörperpräparaten, die ein Protoxin enthalten, das von besagten transformierten *Bacillus* Zellen produziert wird.

Die Formulierungen, d.h. die die transformierten lebenden oder toten *Bacillus*-Zellen oder Mischungen davon sowie die von besagten transformierten *Bacillus*-Zellen produzierten Toxin-Proteine und gegebenenfalls feste oder flüssige Hilfsmittel enthaltenden Mittel oder Zubereitungen werden in bekannter Weise hergestellt, z.B. durch inniges Vermischen der transformierten Zellen und/oder Toxin-Proteine mit festen Trägerstoffen und gegebenenfalls oberflächenaktiven Verbindungen (Tensiden).

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen nichtionogene, kation- und/oder anionaktive Tenside mit guten Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder unsubstituierte oder substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurin-salze zu erwähnen, wie z.B. das Natriumsalz der cis-2-(methyl-9-octadecenylamino)-ethansulfonsäure (Gehalt in Formulierungen vorzugsweise etwa 3 %).

Häufiger werden jedoch sogenannte synthetische Tenside verwendet, insbesondere Fettsulfonate, Fettsulfate, sulfonierte Benzimidazolderivate oder Alkylarylsulfonate oder Fett-Alkohole, wie z.B. 2,4,7,9-tetramethyl-5-decin-4,7-diol (Gehalt in Formulierungen vorzugsweise bei etwa 2 %).

Die Fettsulfonate oder -sulfate liegen in der Regel als Alkali-, Erdalkali- oder gegebenenfalls unsubstituierte oder substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylsulfats oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4 bis 14)-ethylenoxid-Adduktes in Frage.

Als nicht-ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nicht-ionische Tenside sind die wasserlöslichen, 20 bis 250 Ethylenglykolethergruppen und 10 bis 100 Propylenglykolethergruppen enthaltenden Polyethylenoxid-Addukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nicht-ionischer Tenside seien Nonylphenolpolyethoxyethanole, Ricinusölpolyglykolether, Polypropylen/Polyethylenoxid-Addukte, Tributylphenoxypolyethoxyethanol, Polyethylenglykol und Octylphenoxypolyethoxyethanol erwähnt. Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten unsubstituierte oder halogenierte Nieder-Alkyl-, -Benzyl-oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die in der Formulierungstechnik gebräuchlichen Tenside sind u.a. in folgenden Publikationen beschrieben: 1986 International McCutcheon's Emulsifiers & Detergents, The Manufacturing Confectioner Publishing Co., Glen Rock, NJ, USA ; Helmut Stache "Tensid-Taschenbuch" Carl Hanser-Verlag München/Wien 1981.

Die agrochemischen Mittel enthalten in der Regel 0,1 bis 99 %, insbesondere 0,1 bis 95 %, der transformierten, lebenden oder toten *Bacillus*-Zellen oder Mischungen davon bzw. der von besagten transformierten *Bacillus*-Zellen produzierten Toxin-Proteinen, 99,9 bis 1 %, insbesondere 99,8 bis 5 %, eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Formulierungen.

Solche Mittel können noch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die transformierten lebenden oder toten *Bacillus*-Zellen oder Mischungen davon, welche die rekombinanten, modifizierten CryIA(b)-Gene aus *B-thuringiensis* enthalten, sowie die von besagten transformierten *Bacillus*-Zellen produzierten temperaturstabilen Protoxin-Proteine selbst sind hervorragend für die Bekämpfung von Schadinsekten geeignet und bilden daher einen weiteren Gegenstand der vorliegenden Erfindung.

Vorzugsweise sind dabei pflanzenzerstörende Insekten der Ordnung *Lepidoptera* zu nennen, insbesondere solche der Gattungen *Pieris, Heliothis, Spodoptera, Lymanthria* und *Plutella,* wie beispielsweise *Pieris brassicae, Heliothis virescens, Heliothis zea, Spodoptera littoralis, Spodoptera frugiperda, Lymanthria dispar und Plutella xylostella.*

Die Aufwandmengen, in denen die *Bacillus*-Zellen bzw. die von diesen produzierten Toxin-Proteine eingesetzt werden, hängen von den jeweiligen Bedingungen ab, wie beispielsweise den Witterungsverhältnissen, der Bodenbeschaffenheit, dem Pflanzenwachstum und dem Applikationszeitpunkt.

**Formulierungsbeispiele für *B. thuringiensis*-Toxin-enthaltendes Material**

Bei den folgenden Formulierungsbeispielen sind unter dem Begriff "*Bacillus*-Zellen" solche *B. thuringiensis* und/oder *B. cereus*-Zellen zu verstehen, die ein erfindungsgemässes rekombinantes CryIA(b)-Gen aus *B. thuringiensis* enthalten. (Bei den Angaben handelt es sich durchgehend um Gewichtsprozente.)

| F1. Granulate | a) | b) |
|---|---|---|
| *Bacillus*-Zellen und/oder von diesen produziertes Toxin-Protein | 5 % | 10 % |
| Kaolin | 94 % | - |
| Hochdisperse Kieselsäure | 1% | - |
| Attapulgit | - | 90 % |

Die *Bacillus*-Zellen und/oder von diesen produziertes Toxin-Protein werden zunächst in Methylenchlorid suspendiert, anschliessend wird die Suspension auf das Trägermaterial aufgesprüht und danach das Suspendierungsagens im Vakuum verdampft.

| F2. Stäubemittel | a) | b) |
|---|---|---|
| *Bacillus*-Zellen und/oder von diesen produziertes Toxin-Protein | 2 % | 5 % |
| Hochdisperse Kieselsäure | 1 % | 5 % |
| Talkum | 97 % | - |
| Kaolin | - | 90 % |

Gebrauchsfertige Stäubemittel erhält man durch inniges Vermischen der Trägerstoffe mit den *Bacillus*-Zel-

len und/oder dem von diesen produzierten Toxin-Protein.

| F3. Spritzpulver | a) | b) | c) |
|---|---|---|---|
| *Bacillus*-Zellen und/oder von diesen produziertes Toxin-Protein | 25 % | 50 % | 75 % |
| Natrium-Ligninsulfonat | 5 % | 5 % | - |
| Natrium-Laurylsulfat | 3 % | - | 5 % |
| Natrium-Diisopropylnaphthalinsulfonat | - | 6% | 10% |
| Octylphenolpolyethylenglykolether (7-8 Mole Ethylenoxid | - | 2 % | - |
| Hochdisperse Kieselsäure | 5 % | 10 % | 10 % |
| Kaolin | 62 % | 27 % | - |

Die *Bacillus*-Zellen und/oder von diesen produziertes Toxin-Protein werden sorgfältig mit den Zusatzstoffen vermischt und das erhaltene Gemisch anschliessend in einer geeigneten Mühle gut vermahlen. Man erhält Spritzpulver, die sich mit Wasser zu Suspensionen jeder gewünschten Konzentration verdünnen lassen.

| F4. Extruder Granulate | |
|---|---|
| *Bacillus*-Zellen und/oder von diesen produziertes Toxin-Protein | 10 % |
| Natrium-Ligninsulfonat | 2 % |
| Carboxymethylcellulose | 1 % |
| Kaolin | 87 % |

Die *Bacillus*-Zellen und/oder von diesen produziertes Toxin-Protein werden mit den Hilfsstoffen gemischt, sorgfältig vermahlen und mit Wasser angefeuchtet. Dieses Gemisch wird extrudiert und anschliessend in Luftstrom getrocknet.

| F5. Umhüllungs-Granulat | |
|---|---|
| *Bacillus*-Zellen und/oder von diesen produziertes Toxin-Protein | 3 % |
| Polyethylenglykol 200 | 3 % |
| Kaolin | 94 % |

Die homogen vermischten *Bacillus*-Zellen und/oder von diesen produziertes Toxin-Protein werden in einem Mischer auf das mit Polyethylenglykol angefeuchtete Kaolin gleichmässig aufgetragen. Auf diese Weise erhält man staubfreie Umhüllungs-Granulate.

F6. Suspensions-Konzentrat

| | |
|---|---|
| *Bacillus*-Zellen und/oder von diesen produziertes Toxin-Protein | 40 % |
| Ethylenglykol | 10 % |
| Nonylphenolpolyethylenglykol (15 Mole Ethylenoxid) | 6 % |
| Alkylbenzolsulfonsäuretriethanolaminsalz* | 3 % |
| Carboxymethylcellulose | 1 % |
| Silikonöl in Form einer 75 %igen wässrigen Emulsion | 0,1 % |
| Wasser | 39 % |

* Alkyl ist vorzugsweise linear mit 10 bis 14, insbesondere 12-14 Kohlenstoffatomen wie beispielsweise n-Dodecylbenzolsulfonsäuretriethanolaminsalz.

Die homogen vermischten *Bacillus*-Zellen und/oder von diesen produziertes Toxin-Protein werden mit den Zusatzstoffen innig vermischt. Man erhält so ein Suspensionkonzentrat, aus welchem durch Verdünnen mit Wasser Suspensionen jeder gewünschten Konzentration hergestellt werden können.

## Allgemeine rekombinante DNA-Techniken

Da viele der in dieser Erfindung genutzten rekombinanten DNA-Techniken Routine für den Fachmann sind, wird im folgenden eine kurze Beschreibung der allgemein gebräuchlichen Techniken gegeben, sodass auf diese allgemeinen Angaben im konkreten Ausführungsbeispiel verzichtet werden kann. Sofern nicht ausdrücklich darauf hingewiesen wird, sind alle diese Verfahren in der Referenz von Maniatis *et al* (1982) beschrieben.

A. Schneiden mit Restriktionsendonukleasen

Typischerweise sind etwa 50 µg/ml bis 500 µg/ml DNA in dem Reaktionsansatz enthalten, vorzugsweise in einer vom Hersteller empfohlenen Pufferlösung. Für jedes µg DNA werden 2 bis 5 Einheiten von Restriktionsendonukleasen hinzugefügt und der Reaktionsansatz bei der vom Hersteller empfohlenen Temperatur für eine bis drei Stunden inkubiert. Die Reaktion wird durch 10 minütiges Erhitzen auf 65°C oder durch Extraktion mit Phenol, gefolgt durch Präzipitation der DNA mit Ethanol, beendet. Diese Technik wird auch auf den Seiten 104 bis 106 der Maniatis *et al* (1982)-Referenz beschrieben.

B. Behandlung der DNA mit Polymerase, um glatte Enden zu erzeugen

50 µg/ml bis 500 µg/ml DNA-Fragmente werden zu einem Reaktionsansatz hinzugefügt, vorzugsweise in einem vom Hersteller empfohlenen Puffer. Der Reaktionsansatz enthält alle vier Desoxynukleotidtriphosphate in Konzentrationen von 0.2 mM. Nach Zusatz einer geeigneten DNA-Polymerase erfolgt die Reaktion während 30 Minuten bei 15°C und wird dann durch 10 minütiges Erhitzen auf 65°C beendet.

Für Fragmente, die durch Schneiden mit Restriktionsendonukleasen erhalten werden, welche 5'-überstehende Enden erzeugen, wie EcoRI und BamHI , wird das grosse Fragment, oder Klenow-Fragment, der DNA-Polymerase verwendet. Für Fragmente, die durch Endonukleasen erhalten werden, welche 3'-überstehende Enden erzeugen, wie PstI und SacI, wird die T4-DNA-Polymerase verwendet. Die Verwendung dieser beiden Enzyme wird auf den Seiten 113 bis 121 der Maniatis *et al* (1982)-Referenz beschrieben.

C. Agarose-Gelelektrophorese und Reinigung der DNA-Fragmenten von Gelverunreinigungen.

Die Agarose-Gelelektrophorese wird vorzugsweise in einer horizontalen Apparatur durchgeführt, wie dies auf den Seiten 150 bis 163 der Maniatis *et al* (1982)-Referenz beschrieben ist. Der verwendete Puffer entspricht dem dort beschriebenen Tris-Borat- oder Tris-Acetatpuffer. Die DNA-Fragmente können dann beispielsweise mit Hilfe von 0.5 µg/ml Ethidiumbromid angegefärbt werden, das entweder im Gel- oder im Tankpuffer während der Elektrophorese vorhanden ist oder aber wahlweise erst nach Beendigung der Elektrophorese hinzugefügt wird. Die DNA wird durch Beleuchtung mit langwelligem Ultraviolettlicht sichtbar gemacht.

Wenn die Fragmente vom Gel abgetrennt werden sollen, wird vorteilhafterweise eine Agarose verwendet, die bei niedriger Temperatur geliert und die z.B. von Sigma Chemical, St. Louis, Missouri, bezogen werden kann. Nach der Elektrophorese wird das gewünschte Fragment ausgeschnitten, in ein Plastikröhrchen gegeben, etwa 15 Minuten auf 65°C erhitzt, dreimal mit Phenol extrahiert und zweimal mit Ethanol gefällt. Dieses Verfahren ist gegenüber dem von Maniatis *et al* (1982) auf Seite 170 beschriebenen leicht verändert.

Als Alternative kann die DNA auch aus dem Agarosegel mit Hilfe des 'Geneclean Kits' (Bio 101 Inc., La Jolla, CA, US) isoliert werden.

D. Entfernen von 5'-terminalen Phosphaten von DNA-Fragmenten

Während der Plasmidklonierungsschritte vermindert eine Behandlung des Vektorplasmids mit Phosphatase die Rezirkularisation des Vektors [diskutiert auf Seite 13 der Maniatis *et al* (1982)-Referenz]. Nach dem Schneiden der DNA mit der richtigen Restriktionsendonuklease wird eine Einheit alkalische Phosphatase aus dem Darm von Kälbern hinzugefügt, die beispielsweise von Boehringer-Mannheim, Mannheim, erworben werden kann. Die DNA wird eine Stunde bei 37°C inkubiert und anschliessend zweimal mit Phenol extrahiert und mit Ethanol gefällt.

E. Verknüpfen der DNA-Fragmente

Wenn Fragmente mit komplementären kohäsiven Enden miteinander verknüpft werden sollen, werden etwa 100 ng von jedem Fragment in einem Reaktionsgemisch von 20 µl bis 40 µl mit etwa 0.2 Einheiten T4 DNA-Ligase (z.B. von New England Biolabs) vorzugsweise in einem vom Hersteller empfohlenen Puffer inkubiert. Die Inkubation wird 1 bis 20 Stunden lang bei 15°C durchgeführt. Wenn DNA-Fragmente mit glatten Enden verknüpft werden sollen, werden sie wie oben beschrieben inkubiert, wobei aber die Menge der T4 DNA-Ligase in diesem Fall auf 2 bis 4 Einheiten erhöht wird.

F. Die Transformation von DNA in *E. coli*

*E. coli* Stamm HB101 wird für die meisten Experimente verwendet. DNA kann vorteilhafterweise mit dem Kalziumchloridverfahren, wie es von Maniatis *et al* (1982), Seiten 250 bis 251, beschrieben wurde, in *E.coli* eingeführt werden.

G. Screening von *E. coli* auf Plasmide

Nach der Transformation werden die resultierenden Kolonien von *E. coli* auf das Vorhandensein des gewünschten Plasmids durch ein schnelles Plasmidisolationsverfahren geprüft. Zwei gebräuchliche Verfahren werden auf den Seiten 366 bis 369 der Maniatis *et al* (1982)-Referenz beschrieben.

H. Isolierung von Plasmid-DNA in grossem Massstab

Verfahren zur Isolierung von Plasmiden aus *E. coli* in grossem Massstab werden auf den Seiten 88 bis 94 der Maniatis *et al* (1982)-Referenz beschrieben.

**Medien und Pufferlösungen**

| LB-Medium | [g/l] |
|---|---|
| Trypton | 10 |
| Hefeextrakt | 5 |
| NaCl | 5 |

| Antibiotic Medium Nr. 3 (Difco Laboratories) | [g/l] |
|---|---|
| Rinder-Fleischextrakt | 1.5 |
| Hefeextrakt | 1.5 |
| Pepton | 5 |
| Glucose | 1 |
| NaCl | 3.5 |
| $K_2HPO_4$ | 3.68 |
| $KH_2PO_4$ | 1.32 |

| SCGY-Medium | [g/l] |
|---|---|
| Casaminosäuren | 1 |
| Hefeextrakt | 0.1 |
| Glucose | 5 |
| $K_2HPO_4$ | 14 |
| $KH_2PO_4$ | 6 |
| $Na_3$-Citrat | 1 |
| $(NH_4)_2SO_4$ | 2 |
| $MgSO_4 \cdot 7\ H_2O$ | 0.2 |

| GYS-Medium (Yousten & Rogoff, 1969) | [g/l] |
|---|---|
| Glucose | 1 |
| Hefeextrakt | 2 |
| $(NH_4)_2SO_4$ | 2 |
| $K_2HPO_4$ | 0.5 |
| $MgSO_4 \cdot 7\ H_2O$ | 0.2 |
| $CaCl_2 \cdot 2\ H_2O$ | 0.08 |
| $MnSO_4 \cdot H_2O$ | 0.05 |

pH vor Autoklavieren auf 7.3 einstellen.

| PBS-Puffer | [mM] |
|---|---|
| Saccharose | 400 |
| $MgCl_2$ | 1 |
| Phosphat-Puffer, pH 6.0 | 7 |

| TBST-Puffer | [mM] |
|---|---|
| Tween 20* | 0.05% (w/v) |
| Tris/HCl* (pH 8.0) | 10 |
| NaCl | 150 |

| Puffer High<br>[Maniatis *et al* (1982); Seite 104] | [mM] |
|---|---|
| NaCl | 100 |
| Tris/HCl* (pH 7.5) | 50 |
| $MgCl_2$ | 10 |
| Dithiothreitol | 1 |

| Nick-Translationspuffer (10x) | [mM] |
|---|---|
| Tris/HCl* (pH 7.2) | 500 |
| $MgSO_4$ | 100 |
| Dithiothreitol | 1 |
| Rinderserumalbumin (BSA Pentax Fraktion V) | 500 µg/ml |

*Tween 20 Polyethoxysorbitanlaurat

*Tris/HCl α,α,α-Tris(hydroxymethyl)methylaminohydrochlorid

**Nichtlimitierende Ausführungsbeispiele**

1. Konstruktion von Plasmid pXI93

Plasmid pK36 :
Das im Rahmen dieser Erfindung für die Einschleusung und Expression in *B. thuringiensis* bzw. *B. cereus* verwendete CryIA(b)-Gen, das für ein kurhd1 delta-Endotoxin-Protein kodiert, stammt aus dem Plasmid pK36 (=pXI36), das mit Datum vom 4. März 1986 bei der als Internationale Hinterlegungsstelle anerkannten Deutschen Sammlung von Mikroorganismen, BRD, entsprechend den Anforderungen des Budapester Vertrages für die Internationale Anerkennung der Hinterlegung von Mikoorganismen zum Zwecke der Patentierung unter der Hinterlegungsnummer DSM 3668 hinterlegt wurde.

Eine detallierte Beschreibung der Verfahren zur Identifizierung und Isolierung der δ-Endotoxin-Gene sowie

der Konstruktion des Plasmids pK36 ist in der Europäischen Patentanmeldung EP-A 0238 441 enthalten und in Form einer Referenz ein Bestandteil der vorliegenden Erfindung.

Plasmid pXI61 :

Für die Konstruktion eines potenten bifunktionellen Vektors wird zunächst das grosse EcoRI-Fragment von pBC16, einem natürlich vorkommenden Plasmid aus *Bacillus cereus*, mit Hilfe von T4DNA-Ligase in die EcoRI-Stelle des Plasmids pUC8 ([28]Vieira J und Messing J, 1982) eingespleisst. Anschliessend werden *E. coli*-Zellen mit diesem Konstrukt transformiert. Eine mit Hilfe einer Restriktionsanalyse als korrekt erkannte Konstruktion wird pXI 62 genannt.

Es folgt die Entfernung der distal der pUC8 Polylinkerregion gelegenen EcoRI Schnittstelle. Durch eine partielle EcoRI-Verdauung wird pXI 62 linearisiert. Die kohäsiven EcoRI-Enden werden mit Klenow-Polymerase aufgefüllt und mit T4 DNA-Ligase wieder zusammengefügt. Nach Transformation in *E. coli* wird eine mit Hilfe einer Restriktionsanalyse als richtig erkannte Konstruktion ausgewählt und pXI 61 genannt.

Diese Konstruktion lässt sich mit Hilfe der in Beispiel 7 beschriebenen Transformationsmethode direkt in *B. thuringiensis* HD1cryB transformieren.

Plasmid pXI93 :

pK36 Plasmid DNA wird mit den Restriktionsenzymen PstI und BamHI komplett verdaut und das 4.3 Kb umfassende Fragment, das das Kurhd1 δ-Endotoxingen enthält, aus einem Agarosegel isoliert. Dieses Fragment wird dann in pXI61 eingespleisst, welches zuvor mit PstI und BmHI verdaut und mit alkalischer Phosphatase aus dem Kälberdarm behandelt wurde. Nach der Transformation von *E. coli* HB 101 wird ein mit Hilfe einer Restriktionsanalyse als korrekt erkannte Konstruktion isoliert, welche die Bezeichnung pXI93 erhält. Eine detaillierte Beschreibung der Verfahren zur Konstruktion des Plasmids pXI93 ist in der Europäischen Patentanmeldung EP-A 0 342 633 enthalten. Darüberhinaus sind die Plasmide pXI61 und pXI93 bei der als Internationale Hinterlegungsstelle anerkannten'Deutschen Sammlung von Mikroorganismen' (Braunschweig, BRD) gemäss den Bestimmungen des Budapester Vertrages unter der Nummer DSM 4572 (pK61 = pXI61, transformiert in *B. thuringiensis* var. kurstaki HD1 cryB) bzw. DSM 4571 (pK93 = pXI93, transformiert in *B. thuringiensis* var. kurstaki HD1 cryB) und DSM 4573 (pK93 = pXI93, transformiert in *B. cereus* 569 K) hinterlegt.

2. Verdauung der pXI93 DNA

Die Verdauung von 5 µg pXI93 Plasmid-DNA mit dem Restriktionsenzym KpnI wird gemäss den Vorschriften des Herstellers (Boehringer, Mannheim, BRD) durchgeführt. Nach vollständiger Verdauung der DNA wird diese bei niedrigen Temperaturen (-20° C und weniger) unter den bei Maniatis *et al* (1982) [siehe Seite 461] beschriebenen Bedingungen mit Ethanol präzipitiert. Anschliessend wird die DNA in dem von Maniatis *et al* (1982) für eine PstI Verdauung vorgeschlagenen Puffer oder unter Verwendung eines kommerziell erhältlichen Inkubationssets ('incubation set' ; Boehringer, Mannheim ; cat# ; 1082035) gemäss den Angaben des Herstellers resuspendiert und dort erneut vollständig verdaut. Nach Beendigung dieses zweiten Verdauungsschrittes wird das grosse DNA-Fragment auf einem Agarosegel von den übrigen Fragmenten abgetrennt und mit Hilfe einer Elektroelution isoliert.

3. Konstruktion von Plasmid pXI54

3.1 pHD73 : Die aus *Bacillus thuringiensis* subsp. *kurstaki* HD73 [der *B.t.* Stamm HD73 ist bei Adang *et al* (1985) beschrieben und kann bei der "Microbial Property Research, USDA, Northem Reg. Res. Center" in Peoria, USA kommerziell bezogen werden unter der Nummer NRRL HD73] erhältliche Gesamt-DNA wird mit dem Restriktionsenzym HindIII vollständig verdaut und anschliessend in den pUC8 Vektor (New England Biolabs) eingespleisst. Ein korrekter Klon, der durch Hybridiserung mit eimem radioaktiv markierten, 726 Bp umfassenden EcoRI Fragment (Position 423-1149 des CryIA(b)-Gens) ermittelt wird, erhält die Bezeichnung pHD73. Ein für obige Hybridisierungsreaktion geeignetes Fragment kann beispielsweise aus dem Plasmid pXI36 gewonnen werden. Dies kann dann z.B. unter Verwendung eines kommerziell erhältlichen Markierungskits ('oligolabelling kit' ; Pharmacia cat# ; 27-9250-01) radioaktiv markiert werden.

Durch Verdauung von pHD73 mit den Restriktionsenzymen SpeI und HindIII unter den vom Hersteller empfohlenen Bedingungen [Puffer mit hoher Ionenstärke,'Puffer High', Maniatis *et al* (1982), Seite 104] erhält man ein entsprechendes SpeI-HindIII Fragment, auf welchem sich das 5'-Ende des CryIA(c)-Gens [DNA Position 564 bis 3212 in dem bei Adang *et al* (1985) beschriebenen Gen] befindet. Das korrekte, 2648 Bp umfassende Fragment wird dann mit Hilfe einer Gelelektrophorese abgetrennt und durch Elektroelution aus dem Agarosegel isoliert.

3.2 pXI36 : Im Plasmid pXI36 (siehe Beispiel 1) wird die HindIII Erkennungsstelle innerhalb der Polylinkerregion eliminiert, indem besagtes Plasmid zunächst mit Hind III verdaut und die auf diese Weise gebildeten kohäsiven Enden anschliessend mit dNTP [der Reaktionsansatz enthält alle vier Desoxynukleotidtriphosphate] unter Zugabe des Klenow-Fragments der *E.coli* Polymerase I in einem Nick-Translationspuffer aufgefüllt werden [siehe Maniatis *et al* (1982), Seite 394].

Die gebildeten Fragmente, die nunmehr glätte Enden aufweisen werden in einer Ligasereaktion miteinander verknüpft, in *E.coli* HB101 transformiert und mittels einer Restriktionsanalyse analysiert. Ein geeigneter Klon, bei dem die HindIII Erkennungsstelle innerhalb der Polylinkerregion von Plasmid pXI36 eliminiert ist, wird dann mit SpeI und HindIII verdaut und das grosse Fragment mit Hilfe einer Gelelektrophorese abgetrennt und durch Elektroelution aus dem Agarosegel isoliert.

3.3 pXI 54 : Anschliessend wird das zuvor aus pHD73 isolierte SpeI-HindIII Fragment (vergl. 3.1) mit dem grossen Fragment aus pXI36 (vergl. 3.2) mit Hilfe von T4 DNA-Ligase verknüpft und unter den bei Maniatis *et al* (1982) angegebenen Bedingungen (vergl. Seite 250) in einen geeigneten *E.coli* Stamm (z.B. E.coli HB101 oder DH1) einkloniert. Ein korrekter Klon, der mit Hilfe einer Restriktionsanalyse ermittelt werden kann, erhält die Bezeichnung pXI54.

## 4. Isolierung eines 1460 Bp umfassenden HindIII-PstI Fragmentes aus pXI36

Das 3'-Ende des CryIA(b)-Gens befindet sich auf einem 1460 Bp umfassenden HindIII-PstI Fragement von Plasmid pXI36 [Position 2899 - 4359 der in EP-A 342 633 wiedergegebenen Sequenz]. 5 µg pXI36 DNA werden mit den Restriktionsenzymen HindIII und PstI gemäss den Angaben des Herstellers (Boehringerm, Mannheim; 'incubation buffer set', cat# 1 882 035) vollständig verdaut. Das 1460 Bp Fragment wird dann über ein Agarosegel abgetrennt.

## 5. Isolierung eines 648 Bp umfassenden KpnI-HindIII Fragmentes aus pXI54

5 µg pXI54 DNA werden unter Standardbedingungen, die vom Hersteller (Boehringer, Mannheim, BRD) vorgegeben sind, mit KpnI und HindIII verdaut. Das 648 Bp Fragment [Position 2564 - 3212 in der bei Adang *et al* (1985) publizierten Sequenz] wird dann über ein Agarosegel abgetrennt.

## 6. Verknüpfung der isolierten Fragmente und Einklonierung in *B. thuringiensis*

Das grosse KpnI-PstI Fragment aus Plasmid pXI93 (vergl. Punkt 2), das 648 Bp umfassende KpnI-PstI Fragment aus dem Plasmid pXI54 enthaltend einen Abschnitt des CryIA(c)-Gens (vergl. Punkt 5) sowie das 1460 Bp umfassende Fragment aus pXI36 (vergl. Punkt 4) werden unter Standardbedingungen miteinander verknüpft und in *E.coli* HB101 einkloniert. Nach Restriktionsanlayse wird ein korrekter Klon isoliert, der die Bezeichnung pXI106 erhält.

Die Sequenz des δ-Endotoxinsgens, welches sich auf Plasmid pXI106 befindet, ist im Prinzip identisch mit der bekannten Sequenz aus Plasmid pXI93. Der einzige Unterschied betrifft den Sequenzabschnitt zwischen den Positionen 2329 (KpnI-Schnittstelle) und 2899 (HindIII-Schnittstelle) im CryIA(b)-Gen [siehe SEQ ID NO: 1] . Diese Fragment ist im Plasmid pXI106 ausgetauscht durch ein entsprechendes KpnI-HindIII Fragment aus dem CryIA(c)-Gen, welches sich dort zwischen den Positionen 2564 (KpnI-Schnittstelle) und 3212 (HindIII-Schnittstelle) befindet [siehe SEQ ID NO : 2].

Dieses pXI106 Plasmid wird dann in den *B.thuringiensis* Stamm cryB transformiert. Als Kontrolle fungiert ein ensprechender *B.thuringiensis* Stamm, der mit Plasmid pXI93 transformiert ist.

## 7. Transformation von pXI106 in *B. thuringiensis* cryB

10 ml eines LB Mediums (Trypton 10 g/l, Hefeextrakt 5 g/l, NaCl 5 g/l) werden mit Sporen von *B. thuringiensis* var. kurstaki HD1cryB (Stahly DP *et al*, 1978) einer plasmidfreien Variante von *B. thuringiensis* var. kurstaki HD1, inokuliert.

Dieser Ansatz wird über Nacht bei einer Temperatur von 27°C auf einer Rundschüttelmaschine bei 50 Upm inkubiert. Danach wird die *B. thuringiensis* Kultur in 100 ml bis 400 ml LB-Medium 100fach verdünnt und bei einer Temperatur von 30°C auf einer Rundschüttelmaschine bei 250 Upm weiter kultiviert, bis eine optische Dichte ($OD_{550}$) von 0.2 erreicht ist.

Die Zellen werden mit Hilfe einer Zentrifugation geerntet und in 1/40 Volumen eines eisgekühlten PBS-Puffers (400 mM Saccharose, 1 mM $MgCl_2$, 7 mM Phosphat-Puffer pH 6.0) suspendiert. Die Zentrifugation und anschliessende Suspension der geernteten *B. thuringiensis*-Zellen in PBS-Puffer wird einmal wiederholt.

Die so vorbehandelten Zellen können dann entweder direkt elektroporiert oder aber nach Zugabe von Glycerin in die Pufferlösung [20% (w/v)], bei -20°C bis -70°C aufbewahrt und zu einem späteren Zeitpunkt verwendet werden.

800 µl Aliquots der eisgekühlten Zellen werden dann in vorgekühlte Küvetten überführt, anschliessend wird 0.2 µg Plasmid DNA (20 µg/ml) zugegeben und der gesamte Ansatz 10 Minuten bei 4°C inkubiert.

Bei Verwendung von tiefgekühltem Zellmaterial wird zunächst ein geeignetes Aliquot gefrorener Zellen in Eis oder bei Raumtemperatur aufgetaut. Die weitere Behandlung erfolgt analog dem Vorgehen bei Verwendung frischen Zellmaterials.

Danach wird die Küvette in eine Elektroporationsapparatur eingebracht und die in der Suspension vorliegenden *B. thuringiensis*-Zellen werden einer Elektroporation unterzogen, indem sie durch einmaliges Entladen eines Kondensators mit Spannungen zwischen 0.1 kV und 2.5 kV beaufschlagt werden.

Der hier verwendete Kondensator weist eine Kapazität von 25 µF auf, die Küvetten haben einen Elektrodenabstand von 0.4 cm, was bei einer Entladung je nach Einstellung zu einer exponentiell abnehmenden Feldstärke mit anfänglichen Spitzenwerten von 0.25 kV/cm bis 6.25 kV/cm führt. Die exponentielle Abklingzeit liegt in einem Bereich zwischen 10 ms und 12 ms.

Für die beschriebenen Elektroporationsversuche kann beispielsweise ein Elektroporationsgerät der Firma Bio Rad verwendet werden ('Gene Pulser Apparatus', # 165-2075, Bio Rad, 1414 Harbour Way South, *Richmond*, CA 94804, USA).

Selbstverständlich ist auch jedes andere geeignete Gerät in dem erfindungsgemässen Verfahren einsetzbar.

Nach einer weiteren 10 minütigen Inkubation bei 4°C wird die Zellsuspension mit 1.2 ml LB Medium verdünnt und 2 Stunden bei einer Temperatur von 30°C auf einer Rundschüttelmaschine bei 250 Upm inkubiert.

Anschliessend werden geeignete Verdünnungen auf LB Agar (LB Medium verfestigt mit Agar, 15 g/l) ausplattiert, der ein für die Selektion des neu erhaltenen Plasmids geeignetes Antibiotikum als Zusatz enthält. Im Falle von pXI106 handelt es sich dabei um das Antibiotikum Tetracyclin, das dem Medium in einer Konzentration von 20 mg/l zugegeben wird.

*Bacillus cereus* Zellen können in gleicher Weise wie *B. thuringiensis* Zellen nach obigem Protokoll transformiert werden.

8. Expression des modifizierten CryIA(b)-Toxins (pXI106) und des Wildtyp-Toxins (pXI93) in *B.thuringiensis* CryB bei 25° C sowie bei 33° C

Zum Animpfen des Sporulationsmediums [GYS-Medium (50 ml) mit Tetracyclin (20 µg/ml)] mit den *B.thuringiensis* Stämmen CryB (pXI106) und CryB (pXI93) werden 0.5 ml einer in einem LB Medium [mit 20 µg/ml Tetracyclin] herangezogenen Übernachtkultur verwendet. Die beiden *B.thuringiensis* Stämme CryB (pXI106) und CryB (pXI93) werden bei einer Kultivierungstemperatur von 25° C bzw. 33° C unter ständiger Bewegung [ca. 250 Upm] in dem Sporulationsmedium (GYS-Medium) bis zum Ende der Sporulationsphase kultiviert.

Im Verlauf er Wachstumsphase wrden ständig Proben gezogen (1.5 ml) und mittels SDS-PAGE analysiert. Die Proben werden ausserdem mikroskopisch auf Anwesenheit von Sporen oder Kristallen hin untersucht.

Nach der Lyse der Zellen werden die Protoxinkristalle zusammen mit den zellulären Bestandteilen abzentrifugiert und anschliessend in einem geeigneten Puffer wieder resuspendiert. Aus dieser Suspension werden dann identische Aliquots entnommen und mit Hilfe einer Polyacrylamid-Gelelektrophorese (7.5% SDS-PAGE) aufgetrennt. Die Protoxin-haltigen Fraktionen werden aus dem Gel eluiert und in PBS-Puffer resuspendiert.

9. ELISA Assay zur quantitativen Bestimmung der pXI106 und pXI93 Protoxine

Das detaillierte Protokoll zu diesem Immunassay ist in "Antibodies : A Laboratory Manual", eds. Harlow E and Lane D ; Cold Spring Harbor Laboratories, (1988) beschrieben. Bei den in diesem Immunassay verwendeten Antikörpern handelt es sich zum einen um spezifisch gegen *B.t.* Protoxin gerichtete monoklonale Antikörper sowie um Peroxidase-markierte anti-Maus IgGs aus Kaninchen, die z.B. bei der DIANAVA GmbH [Hamburg, BRD ; cat.# 315-035-045] erhältlich sind.

9.1 Herstellung monoklonaler Antikörper gegen *B.thuringiensis* Protoxin

Die Herstellung monoklonaler Antikörper gegen δ-Endotoxin aus *Bacillus thuringiensis* var. *kurstaki* HD1 wird analog des bei Huber-Luka č *et al* (1986) beschriebenen Verfahrens durchgeführt.

Bei den für die Antikörperherstellung verwendeten Hybridoma-Zellen handelt es sich um Fusionsprodukte von Sp2/O-Ag Myelomazellen [beschreiben bei Shulman *et al* (1978), diese Zellinie kann bei der 'American Type Culture Collection' in Rockville, Maryland, USA kommerziell bezogen werden] und Milzlymphozyten von

**Literaturverzeichnis**

Antibodies : A Laboratory Manual", eds. Harlow E and Lane D ; Cold Spring Harbor Laboratories, (1988)
Adang *et al*, Gene, 36 : 289-300 (1985)
Bevan *et al*, Nature, 304 : 184-187 (1983)
Bolivar *et al*, Gene, 2 : 95-113 (1977)
Geiser *et al*, Gene, 48 : 109-118, (1986)
Hinnen *et al*, Proc. Natl. Acad. Sci., USA, 75 : 1929-1933 (1978)
Höfte and Whiteley, Microbiol. Rew., 53(2) : 242-255 (1989)
Huber-Lukač *et al*, Infect. Immunol., 54 : 228-232 (1986)
Kronstad JW and Whiteley, J. Bacteriol., 160 : 95-102 (1984)
Maniatis *et al*, Molecular Cloning, A Laboratory Manual, Cold Spring Harbor Laboratory, Cold Spring Harbor, USA, (1982)
McLinden *et al*, Appl. Environ. Microbiol., 50 : 623-628 (1985)
Norrander *et al*, Gene, 26 : 101-104 (1983)
Shulman *et al*, Nature, 276 : 269 (1978)
Schurter *et al*, Mol. Gen. Genet., 218 : 177-181 (1989)
Stahly DP *et al*, Biochem. Biophys. Res. Comm., 84 : 581-588 (1978)
Wong *et al*, J. Biol. Chem., 258(3) : 1960 (1983)
Young *et al*, Proc. Natl. Acad. Sci., USA, 80 : 1194-1198 (1983)
Youston AA and Rogoff MH, J. Bacteriol., 100 : 1229- 1236, 1969
1986 International McCutcheon's Emulsifiers & Detergents, The Manufacturing Confectioner Publishing Co., Glen Rock, NJ, USA
Helmut Stache "Tensid-Taschenbuch" Carl Hanser-Verlag München/Wien 1981.

Patentliteratur
EP-A 238 441
EP-A 342 633
US-P 4,237,224
US-P 4,468,464
US-P 4,683,202

**SEQ ID NO : 1**

**ART DER SEQUENZ** : Nucleotid-Sequenz
**SEQUENZLÄNGE** : 570 Basen
**STRANGFORM** : Einzelstrang
**ART DES MOLEKÜLS** : CryIA(b)-Gen [Position 2329-2899]
**URSPRÜNGLICHE HERKUNFT**
**ORGANISMUS** : *Bacillus thuringiensis* subsp. *kurstaki* HD1
**UNMITTELBARE EXPERIMENTELLE HERKUNFT**
**ORGANISMUS** : *E.coli* HB101 (pK36) [DSM 3667]

BALB/c Mäusen, die zuvor mit δ-Endotoxin und *Bacillus thuringiensis* var. *kurstaki* HD1 immunisiert wurden.

Auf diese Weise können monoklonale Antikörper erhalten werden, die spezifisch gegen das δ-Endotoxin von *Bacillus thuringiensis* var. *kurstaki* HD1 gerichtet sind.

Es können aber durchaus auch andere monoklonale oder aber polyklonale Antikörper in dem sich anschliessenden Immunassay eingesetzt werden.

9.2 ELISA-Assay

(1) Aliquots der in Abschnitt 8 teilweise gereinigten Antigenprobe werden an Mikrotiterplatten gebunden, indem in jede Vertiefung 50 µl der Antigen-haltigen Suspension (in PBS) gegeben werden ;

(2) Es folgt eine 2 stündige Inkubation bei Raumtemperatur in feuchter Atmosphäre ;

(3) Die Platten werden 2x mit PBS gewaschen ;

(4) Die verbliebenen freien Proteinbindungsstellen auf der Platte werden mit Hilfe eines Blockierungs-Puffers abgesättigt. Dazu werden die Vertiefungen mit einem 3% BSA/PBS Puffer, der 0.02% Natriumazid enthält, aufgefüllt und 2 Stunden bei Raumtemperatur und in feuchter Atmosphäre inkubiert ;

(5) Die Platten werden 2x mit PBS geswaschen ;

(6) In jede der gemäss obigem Protokoll vorbehandelten Vertiefungen werden 50 µl einer Antikörper-haltigen Lösung (monoklonaler Antikörper gegen δ-Endotoxin aus *Bacillus thuringiensis* var. *kurstaki* HD1) zugegeben und der gesamte Ansatz wird 2 Stunden bei Raumtemperatur und in feuchter Atmosphäre inkubiert ;

(7) Die nicht gebundenen Antikörper werden durch 2 maliges Waschen mit PBS entfernt ;

(8) Anschliessend werden in jede Vertiefung 50 µl eines zweiten, markierten Antikörpers (Peroxidase-markierte anti-Maus IgGs aus Kaninchen) zugegeben. Der gesamte Ansatz wird 2 Stunden bei Raumtemperatur und in feuchter Atmosphäre inkubiert. Um eine genaue quantitative Bestimmung zu gewährleisten, sollte der zweite Antikörper im Überschuss vorliegen.

(9) Die nicht gebundenen Antikörper werden durch 4 maliges Waschen mit PBS entfernt ;

(10) Nach dem letzten Waschvorgang wird in jede der Vertiefungen 50 µl der Substratlösung [0.1 mg 3',3',5',5'-Tetramethylbenzidin (TMB) in 0.1 ml Dimethylsulfoxid gelöst. Zu dieser Lösung werden 9.9 ml 0.1M Natriumacetat (pH 6.0) zugegeben, das Ganze wird anschliessend durch einen Whatman No. 1 oder einen vergleichbaren Filter filtriert und mit Wasserstoffperoxid auf eine Endkonzentration von 0.01% eingestellt.] hinzugefügt.

(11) Die Inkubationszeit beträgt 10 bis 30 Minuten bei Raumtemperatur. Die Positiven haben ein fahlblaues Aussehen ;

(12) Nach Zugabe von 50 µl einer 1M $H_2SO_4$ erscheinen die Positiven leuchtend gelb ;

(13) Die qunatitative Bestimmung erfolgt bei 450 nm.

Die Ergebnisse sind in Tabelle 1 wiedergegeben.

Bei einer Kultivierungstemperatur von 25° C sind sowohl die pXI106 als auch die pXI93 Genprodukte stabil und damit in der Nährlösung nachweisbar.

Bei einer Kultivierungstemperatur von 33°C dagegen findet man fast ausschliesslich modifiziertes CryIA(b)-Toxin, während das Genprodukt von Plasmid pXI93 in einer ca. 30fach niedrigeren Konzentration vörliegt.

Tabelle 1 : Konzentration der Genprodukte von pXI93 [CryIA(b)-Gen] und pXI106 [modifiziertes CryIA(b)-Gen] in der Sporulationskultur bei 25°C bzw. 33°C.

| | **Temperatur** | |
|---|---|---|
| **Plasmid** | **25°C** | **33°C** |
| **pXI93 [CryIA(b)-Gen]** | **14.8 *** | **0.53*** |
| **pXI106 [modifiziertes CryIA(b)-Gen]** | **17.1 *** | **17.6*** |

*** diese Angaben beziehen sich auf µg Toxin/ml Sporulationskultur**

```
      K
      p
      n
      I
  TGGGTACC TTTGATGAGT GCTATCCAAC GTATTTATAT CAAAAAATAG  2370
ATGAGTCGAA ATTAAAAGCC TATACCCGTT ACCAATTAAG AGGGTATATC  2420
GAAGATAGTC AAGACTTAGA AATCTATTTA ATTCGCTACA ATGCCAAACA  2470
CGAAACAGTA AATGTGCCAG GTACGGGTTC CTTATGGCCG CTTTCAGCCC  2520
CAAGTCCAAT CGGAAAATGT GCCCATCATT CCCATCATTT CTCCTTGGAC  2570
ATTGATGTTG GATGTACAGA CTTAAATGAG GACTTAGGTG TATGGGTGAT  2620
ATTCAAGATT AAGACGCAAG ATGGCCATGC AAGACTAGGA AATCTAGAAT  2670
TTCTCGAAGA GAAACCATTA GTAGGAGAAG CACTAGCTCG TGTGAAAAGA  2720
GCGGAGAAAA AATGGAGAGA CAAACGTGAA AAATTGGAAT GGGAAACAAA  2770
TATTGTTTAT AAAGAGGCAA AAGAATCTGT AGATGCTTTA TTTGTAAACT  2820
CTCAATATGA TAGATTACAA GCGGATACCA ACATCGCGAT GATTCATGCG  2870
GCAGATAAAC GCGTTCATAG CATTCGAGAA GCTTA                 2905
                              H
                              i
                              n
                              d
                              I
                              I
                              I
```

**SEQ ID NO : 2**

**ART DER SEQUENZ** : Nucleotid-Sequenz
**SEQUENZLÄNGE** : 648 Basen

**STRANGFORM** : Doppelstrang
**ART DES MOLEKÜLS** : CryIA(c)-Gen [Position 2564-3212]

**URSPRÜNGLICHE HERKUNFT**
**ORGANISMUS** : *Bacillus thuringiensis* subsp. *kurstaki* HD73
**UNMITTELBARE EXPERIMENTELLE HERKUNFT**
**ORGANISMUS** : pHD73

```
                   K
                   p
                   n
                   I
           AG GTACCTTTGA TGAGTGCTAT CCAACATATT TGTATCAAAA 2600
           TC CATGGAAACT ACTCACGATA GGTTGTATAA ACATAGTTTT

AATCGATGAA TCAAAATTAA AAGCCTTTAC CCGTTATCAA TTAAGAGGGT 2650
TTAGCTACTT AGTTTTAATT TTCGGAAATG GGCAATAGTT AATTCTCCCA

ATATCGAAGA TAGTCAAGAC TTAGAAATCT ATTTAATTCG CTACAATGCA 2700
TATAGCTTCT ATCAGTTCTG AATCTTTAGA TAAATTAAGC GATGTTACGT

AAACATGAAA CAGTAAATGT GCCAGGTACG GGTTCCTTAT GGCCGCTTTC 2750
TTTGTACTTT GTCATTTACA CGGTCCATGC CCAAGGAATA CCGGCGAAAG

AGCCCAAAGT CCAATCGGAA AGTGTGGAGA GCCGAATCGA TGCGCGCCAC 2800
TCGGGTTTCA GGTTAGCCTT TCACACCTCT CGGCTTAGCT ACGCGCGGTG

ACCTTGAATG GAATCCTGAC TTAGATTGTT CGTGTAGGGA TGGAGAAAAG 2850
TGGAACTTAC CTTAGGACTG AATCTAACAA GCACATCCCT ACCTCTTTTC

TGTGCCCATC ATTCGCATCA TTTCTCCTTA GACATTGATG TAGGATGTAC 2900
ACACGGGTAG TAAGCGTAGT AAAGAGGAAT CTGTAACTAC ATCCTACATG

AGACTTAAAT GAGGACCTAG GTGTATGGGT GATCTTTAAG ATTAAGACGC 2950
TCTGAATTTA CTCCTGGATC CACATACCCA CTAGAAATTC TAATTCTGCG

AAGATGGGCA CGCAAGACTA GGGAATCTAG AGTTTCTCGA AGAGAAACCA 3000
TTCTACCCGT GCGTTCTGAT CCCTTAGATC TCAAAGAGCT TCTCTTTGGT

TTAGTAGGAG AAGCGCTAGC TCGTGTGAAA AGAGCGGAGA AAAAATGGAG 3050
AATCATCCTC TTCGCGATCG AGCACACTTT TCTCGCCTCT TTTTTACCTC

AGACAAACGT GAAAAATTGG AATGGGAAAC AAATATCGTT TATAAAGAGG 3100
TCTGTTTGCA CTTTTTAACC TTACCCTTTG TTTATAGCAA ATATTTCTCC

CAAAAGAATC TGTAGATGCT TTATTTGTAA ACTCTCAATA TGATCAATTA 3150
GTTTTCTTAG ACATCTACGA AATAAACATT TGAGAGTTAT ACTAGTTAAT

CAAGCGGATA CGAATATTGC CATGATTCAT GCGGCAGATA AACGTGTTCA 3200
GTTCGCCTAT GCTTATAACG GTACTAAGTA CGCCGTCTAT TTGCACAAGT

TAGCATTCGA GAAGCTTA                                   3218
ATCGTAAGCT CTTCGAAT
           H
           i
           n
           d
           I
           I
           I
```

**SEQ ID NO : 3**

**ART DER SEQUENZ** : Aminosäure-Sequenz [Sequenzvergleich]

**SEQUENZLÄNGE** : Sequenz (a) : 197 Aminosäuren Sequenz (b) : 223 Aminosäuren

**ART DES MOLEKÜLS** : Protein [δ-Endotoxin]

**URSPRÜNGLICHE HERKUNFT**
**ORGANIMUS** : *Bacillus thuringiensis*
**UNMITTELBARE EXPERIMENTELLE HERKUNFT**
**ORGANISMUS** : Sequenz (a) *Bacillus thuringiensis* CryB (pXI93)
Sequenz (b) *Bacillus thuringiensis* CryB (pXI106)

```
Val Thr Leu Leu Gly Thr Phe Asp Glu Cys Tyr Pro Thr Tyr   733
 |   |   |       |   |   |   |   |   |   |   |   |   |
Val Thr Leu Ser Gly Thr Phe Asp Glu Cys Thy Pro Thr Tyr   733

Leu Tyr Gln Lys Ile Asp Glu Ser Lys Leu Lys Ala Tyr Thr   747
 |   |   |   |   |   |   |   |   |   |   |   |   :   |
Leu Tyr Gln Lys Ile Asp Glu Ser Lys Leu Lys Ala Phe Thr   747

Arg Tyr Gln Leu Arg Gly Tyr Ile Glu Asp Ser Gln Asp Leu   761
 |   |   |   |   |   |   |   |   |   |   |   |   |   |
Arg Tyr Gln Leu Arg Gly Tyr Ile Glu Asp Ser Gln Asp Leu   761

Glu Ile Tyr Leu Ile Arg Tyr Asn Ala Lys His Glu Thr Val   775
 |   |   |   |   |   |   |   |   |   |   |   |   |   |
Glu Ile Tyr Leu Ile Arg Tyr Asn Ala Lys His Glu Thr Val   775
```

```
Asn Val Pro Gly Thr Gly Ser Leu Trp Pro Leu Ser Ala Pro  789
 |   |   |   |   |   |   |   |   |   |   |   |   |
Asn Val Pro Gly Thr Gly Ser Leu Trp Pro Leu Ser Ala Gln  789

Ser Pro Ile Gly ........................................  793
 |   |   |   |
Ser Pro Ile Gly Lys Cys Gly Glu Pro Asn Arg Cys Ala Pro  803

........................................................  793

His Leu Glu Trp Asn Pro Asp Leu Asp Cys Ser Cys Arg Asp  817

....... Lys Cys Ala His His Ser His His Phe Ser Leu Asp  805
         |   |   |   |   |   |   |   |   |   |   |   |
Gly Glu Lys Cys Ala His His Ser His His Phe Ser Leu Asp  831

Ile Asp Val Gly Cys Thr Asp Leu Asn Glu Asp Leu Gly Val  819
 |   |   |   |   |   |   |   |   |   |   |   |   |   |
Ile Asp Val Gly Cys Thr Asp Leu Asp Glu Asp Leu Gly Val  845

Trp Val Ile Phe Lys Ile Lys Thr Gln Asp Gly His Ala Arg  833
 |   |   |   |   |   |   |   |   |   |   |   |   |   |
Trp Val Ile Phe Lys Ile Lys Thr Gln Asp Gly His Ala Arg  859

Leu Gly Asn Leu Glu Phe Leu Glu Glu Lys Pro Leu Val Gly  847
 |   |   |   |   |   |   |   |   |   |   |   |   |   |
Leu Gly Asn Leu Glu Phe Leu Glu Glu Lys Pro Leu Val Gly  873
```

```
Glu Ala Leu Ala Arg Val Lys Arg Ala Glu Lys Lys Trp Arg   861
 |   |   |   |   |   |   |   |   |   |   |   |   |   |
Glu Ala Leu Ala Arg Val Lys Arg Ala Glu Lys Lys Phe Arg   887


Asp Lys Arg Glu Lys Leu Glu Trp Glu Thr Asn Ile Val Tyr   875
 |   |   |   |   |   |   |   |   |   |   |   |   |   |
Asp Lys Arg Glu Lys Leu Glu Phe Glu Thr Asn Ile Val Tyr   901


Lys Glu Ala Lys Glu Ser Val Asp Ala Leu Phe Val Asn Ser   889
 |   |   |   |   |   |   |   |   |   |   |   |   |   |
Lys Glu Ala Lys Glu Ser Val Asp Ala Leu Phe Val Asn Ser   915


Gln Tyr Asp Arg Leu Gln Ala Asp Thr Asn Ile Ala Met Ile   903
 |   |   |       |   |   |   |   |   |   |   |   |   |
Gln Tyr Asp Gln Leu Gln Ala Asp Thr Asn Ile Ala Met Ile   929


His Ala Ala Asp Lys Arg Val His Ser Ile Arg Glu Ala       917
 |   |   |       |   |   |   |   |   |   |   |   |
His Ala Ala Asp Lys Arg Val His Ser Ile Arg Glu Ala       943
```

**SEQ ID NO : 4**

**ART DER SEQUENZ :** Nukleotid-Sequenz
**SEQUENZLÄNGE :** 4438 Basen
**STRANGFORM :** Einzelstrang
**ART DES MOLEKÜLS :** modifiziertes CryIA(b)-Gen
**URSPRÜNGLICHE HERKUNFT**
**ORGANISMUS :** *Bacillus thuringiensis*
**UNMITTELBARE EXPERIMENTELLE HERKUNFT**
**ORGANISMUS :** *Bacillus thuringiensis* CryB (pXI106)

```
  1 GTTAACACCC TGGGTCAAAA ATTGATATTT AGTAAAATTA GTTGCACTTT
 51 GTGCATTTTT TCATAAGATG AGTCATATGT TTTAAATTGT AGTAATGAAA
101 AACAGTATTA TATCATAATG AATTGGTATC TTAATAAAAG AGATGGAGGT
151 AACTTATGGA TAACAATCCG AACATCAATG AATGCATTCC TTATAATTGT
201 TTAAGTAACC CTGAAGTAGA AGTATTAGGT GGAGAAAGAA TAGAAACTGG
251 TTACACCCCA ATCGATATTT CCTTGTCGCT AACGCAATTT CTTTTGAGTG
301 AATTTGTTCC CGGTGCTGGA TTTGTGTTAG GACTAGTTGA TATAATATGG
351 GGAATTTTTG GTCCCTCTCA ATGGGACGCA TTTCTTGTAC AAATTGAACA
401 GTTAATTAAC CAAAGAATAG AAGAATTCGC TAGGAACCAA GCCATTTCTA
451 GATTAGAAGG ACTAAGCAAT CTTTATCAAA TTTACGCAGA ATCTTTTAGA
501 GAGTGGGAAG CAGATCCTAC TAATCCAGCA TTAAGAGAAG AGATGCGTAT
551 TCAATTCAAT GACATGAACA GTGCCCTTAC AACCGCTATT CCTCTTTTTG
601 CAGTTCAAAA TTATCAAGTT CCTCTTTTAT CAGTATATGT TCAAGCTGCA
651 AATTTACATT TATCAGTTTT GAGAGATGTT TCAGTGTTTG GACAAAGGTG
701 GGGATTTGAT GCCGCGACTA TCAATAGTCG TTATAATGAT TTAACTAGGC
751 TTATTGGCAA CTATACAGAT CATGCTGTAC GCTGGTACAA TACGGGATTA
801 GAGCGTGTAT GGGGACCGGA TTCTAGAGAT TGGATAAGAT ATAATCAATT
```

```
851   TAGAAGAGAA TTAACACTAA CTGTATTAGA TATCGTTTCT CTATTTCCGA
901   ACTATGATAG TAGAACGTAT CCAATTCGAA CAGTTTCCCA ATTAACAAGA
951   GAAATTTATA CAAACCCAGT ATTAGAAAAT TTTGATGGTA GTTTTCGAGG
1001  CTCGGCTCAG GGCATAGAAG GAAGTATTAG GAGTCCACAT TTGATGGATA
1051  TACTTAACAG TATAACCATC TATACGGATG CTCATAGAGG AGAATATTAT
1101  TGGTCAGGGC ATCAAATAAT GGCTTCTCCT GTAGGGTTTT CGGGGCCAGA
1151  ATTCACTTTT CCGCTATATG GAACTATGGG AAATGCAGCT CCACAACAAC
1201  GTATTGTTGC TCAACTAGGT CAGGGCGTGT ATAGAACATT ATCGTCCACT
1251  TTATATAGAA GACCTTTTAA TATAGGGATA AATAATCAAC AACTATCTGT
1301  TCTTGACGGG ACAGAATTTG CTTATGGAAC CTCCTCAAAT TTGCCATCCG
1351  CTGTATACAG AAAAAGCGGA ACGGTAGATT CGCTGGATGA AATACCGCCA
1401  CAGAATAACA ACGTGCCACC TAGGCAAGGA TTTAGTCATC GATTAAGCCA
1451  TGTTTCAATG TTTCGTTCAG GCTTTAGTAA TAGTAGTGTA AGTATAATAA
1501  GAGCTCCTAT GTTCTCTTGG ATACATCGTA GTGCTGAATT TAATAATATA
1551  ATTCCTTCAT CACAAATTAC ACAAATACCT TTAACAAAAT CTACTAATCT
1601  TGGCTCTGGA ACTTCTGTCG TTAAAGGACC AGGATTTACA GGAGGAGATA
1651  TTCTTCGAAG AACTTCACCT GGCCAGATTT CAACCTTAAG AGTAAATATT
1701  ACTGCACCAT TATCACAAAG ATATCGGGTA AGAATTCGCT ACGCTTCTAC
1751  CACAAATTTA CAATTCCATA CATCAATTGA CGGAAGACCT ATTAATCAGG
1801  GGAATTTTTC AGCAACTATG AGTAGTGGGA GTAATTTACA GTCCGGAAGC
1851  TTTAGGACTG TAGGTTTTAC TACTCCGTTT AACTTTTCAA ATGGATCAAG
1901  TGTATTTACG TTAAGTGCTC ATGTCTTCAA TTCAGGCAAT GAAGTTTATA
1951  TAGATCGAAT TGAATTTGTT CCGGCAGAAG TAACCTTTGA GGCAGAATAT
2001  GATTTAGAAA GAGCACAAAA GGCGGTGAAT GAGCTGTTTA CTTCTTCCAA
2051  TCAAATCGGG TTAAAAACAG ATGTGACGGA TTATCATATT GATCAAGTAT
2101  CCAATTTAGT TGAGTGTTTA TCTGATGAAT TTTGTCTGGA TGAAAAAAAA
```

2151 GAATTGTCCG AGAAAGTCAA ACATGCGAAG CGACTTAGTG ATGAGCGGAA
2201 TTTACTTCAA GATCCAAACT TTAGAGGGAT CAATAGACAA CTAGACCGTG
2251 GCTGGAGAGG AAGTACGGAT ATTACCATCC AAGGAGGCGA TGACGTATTC
2301 AAAGAGAATT ACGTTACGCT ATTGGGTACC TTTGATGAGT GCTATCCAAC
2351 ATATTTGTAT CAAAAAATCG ATGAATCAAA ATTAAAAGCC TTTACCCGTT
2401 ATCAATTAAG AGGGTATATC GAAGATAGTC AAGACTTAGA AATCTATTTA
2451 ATTCGCTACA ATGCAAAACA TGAAACAGTA AATGTGCCAG GTACGGGTTC
2501 CTTATGGCCG CTTTCAGCCC AAAGTCCAAT CGGAAAGTGT GGAGAGCCGA
2551 ATCGATGCGC GCCACACCTT GAATGGAATC CTGACTTAGA TTGTTCGTGT
2601 AGGGATGGAG AAAAGTGTGC CCATCATTCG CATCATTTCT CCTTAGACAT
2651 TGATGTAGGA TGTACAGACT TAAATGAGGA CCTAGGTGTA TGGGTGATCT
2701 TTAAGATTAA GACGCAAGAT GGGCACGCAA GACTAGGGAA TCTAGAGTTT
2751 CTCGAAGAGA AACCATTAGT AGGAGAAGCG CTAGCTCGTG TGAAAAGAGC
2801 GGAGAAAAAA TGGAGAGACA AACGTGAAAA ATTGGAATGG GAAACAAATA
2851 TCGTTTATAA AGAGGCAAAA GAATCTGTAG ATGCTTTATT TGTAAACTCT
2901 CAATATGATC AATTACAAGC GGATACGAAT ATTGCCATGA TTCATGCGGC
2951 AGATAAACGT GTTCATAGCA TTCGAGAAGC TTATCTGCCT GAGCTGTCTG
3001 TGATTCCGGG TGTCAATGCG GCTATTTTTG AAGAATTAGA AGGGCGTATT
3051 TTCACTGCAT TCTCCCTATA TGATGCGAGA AATGTCATTA AAAATGGTGA
3101 TTTTAATAAT GGCTTATCCT GCTGGAACGT GAAAGGGCAT GTAGATGTAG
3151 AAGAACAAAA CAACCACCGT TCGGTCCTTG TTGTTCCGGA ATGGGAAGCA
3201 GAAGTGTCAC AAGAAGTTCG TGTCTGTCCG GGTCGTGGCT ATATCCTTCG
3251 TGTCACAGCG TACAAGGAGG GATATGGAGA AGGTTGCGTA ACCATTCATG
3301 AGATCGAGAA CAATACAGAC GAACTGAAGT TTAGCAACTG TGTAGAAGAG
3351 GAAGTATATC CAAACAACAC GGTAACGTGT AATGATTATA CTGCGACTCA
3401 AGAAGAATAT GAGGGTACGT ACACTTCTCG TAATCGAGGA TATGACGGAG

3451 CCTATGAAAG CAATTCTTCT GTACCAGCTG ATTATGCATC AGCCTATGAA
3501 GAAAAAGCAT ATACAGATGG ACGAAGAGAC AATCCTTGTG AATCTAACAG
3551 AGGATATGGG GATTACACAC CACTACCAGC TGGCTATGTG ACAAAAGAAT
3601 TAGAGTACTT CCCAGAAACC GATAAGGTAT GGATTGAGAT CGGAGAAACG
3651 GAAGGAACAT TCATCGTGGA CAGCGTGGAA TTACTTCTTA TGGAGGAATA
3701 ATATATGCTT TATAATGTAA GGTGTGCAAA TAAAGAATGA TTACTGACTT
3751 GTATTGACAG ATAAATAAGG AAATTTTTAT ATGAATAAAA AACGGGCATC
3801 ACTCTTAAAA GAATGATGTC CGTTTTTTGT ATGATTTAAC GAGTGATATT
3851 TAAATGTTTT TTTTGCGAAG GCTTTACTTA ACGGGGTACC GCCACATGCC
3901 CATCAACTTA AGAATTTGCA CTACCCCCAA GTGTCAAAAA ACGTTATTCT
3951 TTCTAAAAAG CTAGCTAGAA AGGATGACAT TTTTTATGAA TCTTTCAATT
4001 CAAGATGAAT TACAACTATT TTCTGAAGAG CTGTATCGTC ATTTAACCCC
4051 TTCTCTTTTG GAAGAACTCG CTAAAGAATT AGGTTTTGTA AAAAGAAAAC
4101 GAAAGTTTTC AGGAAATGAA TTAGCTACCA TATGTATCTG GGGCAGTCAA
4151 CGTACAGCGA GTGATTCTCT CGTTCGACTA TGCAGTCAAT TACACGCCGC
4201 CACAGCACTC TTATGAGTCC AGAAGGACTC AATAAACGCT TTGATAAAAA
4251 AGCGGTTGAA TTTTTGAAAT ATATTTTTTC TGCATTATGG AAAAGTAAAC
4301 TTTGTAAAAC ATCAGCCATT TCAAGTGCAG CACTCACGTA TTTTCAACGA
4351 ATCCGTATTT TAGATGCGAC GATTTTCCAA GTACCGAAAC ATTTAGCACA
4401 TGTATATCCT GGGTCAGGTG GTTGTGCACA AACTGCAG

**SEQ ID NO : 5**

**ART DER SEQUENZ** : Aminosäure-Sequenz
**SEQUENZLÄNGE** : 1181 Aminosäuren

**ART DES MOLEKÜLS** : Protein [δ-Endotoxin]

**URSPRÜNGLICHE HERKUNFT**
**ORGANISMUS** : *Bacillus thuringiensis*
**UNMITTELBARE EXPERIMENTELLE HERKUNFT**
**ORGANISMUS** : *Bacillus thuringiensis* CryB (pXI106)

Met Asp Asn Asn Pro Asn Ile Asn Glu Cys Ile Pro Tyr Asn 14

Cys Leu Ser Asn Pro Glu Val Glu Val Leu Gly Gly Glu Arg 28

Ile Glu Thr Gly Tyr Thr Pro Ile Asp Ile Ser Leu Ser Leu 42

Thr Gln Phe Leu Leu Ser Glu Phe Val Pro Gly Ala Gly Phe 56

Val Leu Gly Leu Val Asp Ile Ile Trp Gly Ile Phe Gly Pro 70

Ser Gln Trp Asp Ala Phe Leu Val Gln Ile Glu Gln Leu Ile 84

Asn Gln Arg Ile Glu Glu Phe Ala Arg Asn Gln Ala Ile Ser 98

Arg Leu Glu Gly Leu Ser Asn Leu Tyr Gln Ile Tyr Ala Glu 112

Ser Phe Arg Glu Trp Glu Ala Asp Pro Thr Asn Pro Ala Leu 126

Arg Glu Glu Met Arg Ile Gln Phe Asn Asp Met Asn Ser Ala 140

| | | | | | | | | | | | | | | |
|---|---|---|---|---|---|---|---|---|---|---|---|---|---|---|
| Leu | Thr | Thr | Ala | Ile | Pro | Leu | Phe | Ala | Val | Gln | Asn | Tyr | Gln | 154 |
| Val | Pro | Leu | Leu | Ser | Val | Tyr | Val | Gln | Ala | Ala | Asn | Leu | His | 168 |
| Leu | Ser | Val | Leu | Arg | Asp | Val | Ser | Val | Phe | Gly | Gln | Arg | Trp | 182 |
| Gly | Phe | Asp | Ala | Ala | Thr | Ile | Asn | Ser | Arg | Tyr | Asn | Asp | Leu | 196 |
| Thr | Arg | Leu | Ile | Gly | Asn | Tyr | Thr | Asp | His | Ala | Val | Arg | Trp | 210 |
| Tyr | Asn | Thr | Gly | Leu | Glu | Arg | Val | Trp | Gly | Pro | Asp | Ser | Arg | 224 |
| Asp | Trp | Ile | Arg | Tyr | Asn | Gln | Phe | Arg | Arg | Glu | Leu | Thr | Leu | 238 |
| Thr | Val | Leu | Asp | Ile | Val | Ser | Leu | Phe | Pro | Asn | Tyr | Asp | Ser | 252 |
| Arg | Thr | Tyr | Pro | Ile | Arg | Thr | Val | Ser | Gln | Leu | Thr | Arg | Glu | 266 |
| Ile | Tyr | Thr | Asn | Pro | Val | Leu | Glu | Asn | Phe | Asp | Gly | Ser | Phe | 280 |
| Arg | Gly | Ser | Ala | Gln | Gly | Ile | Glu | Gly | Ser | Ile | Arg | Ser | Pro | 294 |
| His | Leu | Met | Asp | Ile | Leu | Asn | Ser | Ile | Thr | Ile | Tyr | Thr | Asp | 308 |
| Ala | His | Arg | Gly | Glu | Tyr | Tyr | Trp | Ser | Gly | His | Gln | Ile | Met | 322 |
| Ala | Ser | Pro | Val | Gly | Phe | Ser | Gly | Pro | Glu | Phe | Thr | Phe | Pro | 336 |
| Leu | Tyr | Gly | Thr | Met | Gly | Asn | Ala | Ala | Pro | Gln | Gln | Arg | Ile | 350 |
| Val | Ala | Gln | Leu | Gly | Gln | Gly | Val | Tyr | Arg | Thr | Leu | Ser | Ser | 364 |
| Thr | Leu | Tyr | Arg | Arg | Pro | Phe | Asn | Ile | Gly | Ile | Asn | Asn | Gln | 378 |
| Gln | Leu | Ser | Val | Leu | Asp | Gly | Thr | Glu | Phe | Ala | Tyr | Gly | Thr | 392 |

Ser Ser Asn Leu Pro Ser Ala Val Tyr Arg Lys Ser Gly Thr 406

Val Asp Ser Leu Asp Glu Ile Pro Pro Gln Asn Asn Asn Val 420

Pro Pro Arg Gln Gly Phe Ser His Arg Leu Ser His Val Ser 434

Met Phe Arg Ser Gly Phe Ser Asn Ser Ser Val Ser Ile Ile 448

Arg Ala Pro Met Phe Ser Trp Ile His Arg Ser Ala Glu Phe 462

Asn Asn Ile Ile Pro Ser Ser Gln Ile Thr Gln Ile Pro Leu 476

Thr Lys Ser Thr Asn Leu Gly Ser Gly Thr Ser Val Val Lys 490

Gly Pro Gly Phe Thr Gly Gly Asp Ile Leu Arg Arg Thr Ser 504

Pro Gly Gln Ile Ser Thr Leu Arg Val Asn Ile Thr Ala Pro 518

Leu Ser Gln Arg Tyr Arg Val Arg Ile Arg Tyr Ala Ser Thr 532

Thr Asn Leu Gln Phe His Thr Ser Ile Asp Gly Arg Pro Ile 546

Asn Gln Gly Asn Phe Ser Ala Thr Met Ser Ser Gly Ser Asn 560

Leu Gln Ser Gly Ser Phe Arg Thr Val Gly Phe Thr Thr Pro 574

Phe Asn Phe Ser Asn Gly Ser Ser Val Phe Thr Leu Ser Ala 588

His Val Phe Asn Ser Gly Asn Glu Val Tyr Ile Asp Arg Ile 602

Glu Phe Val Pro Ala Glu Val Thr Phe Glu Ala Glu Tyr Asp 616

Leu Glu Arg Ala Gln Lys Ala Val Asn Glu Leu Phe Thr Ser 630

Ser Asn Gln Ile Gly Leu Lys Thr Asp Val Thr Asp Tyr His 644

Ile Asp Gln Val Ser Asn Leu Val Glu Cys Leu Ser Asp Glu 658

Phe Cys Leu Asp Glu Lys Lys Glu Leu Ser Glu Lys Val Lys 672

His Ala Lys Arg Leu Ser Asp Glu Arg Asn Leu Leu Gln Asp 686

Pro Asn Phe Arg Gly Ile Asn Arg Gln Leu Asp Arg Gly Trp 700

Arg Gly Ser Thr Asp Ile Thr Ile Gln Gly Gly Asp Asp Val 714

Phe Lys Glu Asn Tyr Val Thr Leu Ser Gly Thr Phe Asp Glu 728

Cys Tyr Pro Thr Tyr Leu Tyr Gln Lys Ile Asp Glu Ser Lys 742

Leu Lys Ala Phe Thr Arg Tyr Gln Leu Arg Gly Tyr Ile Glu 756

Asp Ser Gln Asp Leu Glu Ile Tyr Leu Ile Arg Tyr Asn Ala 770

Lys His Glu Thr Val Asn Val Pro Gly Thr Gly Ser Leu Trp 784

Pro Leu Ser Ala Gln Ser Pro Ile Gly Lys Cys Gly Glu Pro 798

Asn Arg Cys Ala Pro His Leu Glu Trp Asn Pro Asp Leu Asp 812

Cys Ser Cys Arg Asp Gly Glu Lys Cys Ala His His Ser His 826

His Phe Ser Leu Asp Ile Asp Val Gly Cys Thr Asp Leu Asn 840

Glu Asp Leu Gly Val Trp Val Ile Phe Lys Ile Lys Thr Gln 854

Asp Gly His Ala Arg Leu Gly Asn Leu Glu Phe Leu Glu Glu 868

Lys Pro Leu Val Gly Glu Ala Leu Ala Arg Val Lys Arg Ala 882

Glu Lys Lys Trp Arg Asp Lys Arg Glu Lys Leu Glu Trp Glu 896

Thr Asn Ile Val Tyr Lys Glu Ala Lys Glu Ser Val Asp Ala 910

Leu Phe Val Asn Ser Gln Tyr Asp Gln Leu Gln Ala Asp Thr 924

Asn Ile Ala Met Ile His Ala Ala Asp Lys Arg Val His Ser 938

Ile Arg Glu Ala Tyr Leu Pro Glu Leu Ser Val Ile Pro Gly 952

Val Asn Ala Ala Ile Phe Glu Glu Leu Glu Gly Arg Ile Phe 966

Thr Ala Phe Ser Leu Tyr Asp Ala Arg Asn Val Ile Lys Asn 980

Gly Asp Phe Asn Asn Gly Leu Ser Cys Trp Asn Val Lys Gly 994

His Val Asp Val Glu Glu Gln Asn Asn His Arg Ser Val Leu 1008

Val Val Pro Glu Trp Glu Ala Glu Val Ser Gln Glu Val Arg 1022

Val Cys Pro Gly Arg Gly Tyr Ile Leu Arg Val Thr Ala Tyr 1036

Lys Glu Gly Tyr Gly Glu Gly Cys Val Thr Ile His Glu Ile 1050

Glu Asn Asn Thr Asp Glu Leu Lys Phe Ser Asn Cys Val Glu 1064

Glu Glu Val Tyr Pro Asn Asn Thr Val Thr Cys Asn Asp Tyr 1078

Thr Ala Thr Gln Glu Glu Tyr Glu Gly Thr Tyr Thr Ser Arg 1092

Asn Arg Gly Tyr Asp Gly Ala Tyr Glu Ser Asn Ser Ser Val 1106

Pro Ala Asp Tyr Ala Ser Ala Tyr Glu Glu Lys Ala Tyr Thr 1120

Asp Gly Arg Arg Asp Asn Pro Cys Glu Ser Asn Arg Gly Tyr 1134

Gly Asp Tyr Thr Pro Leu Pro Ala Gly Tyr Val Thr Lys Glu 1148

Leu Glu Tyr Phe Pro Glu Thr Asp Lys Val Trp Ile Glu Ile 1162

Gly Glu Thr Glu Gly Thr Phe Ile Val Asp Ser Val Glu Leu 1176

Leu Leu Met Glu Glu 1181

**Patentansprüche**

1. Modifiziertes CryIA(b)-Gen, bei dem die im C-Terminus des Wildtyp-Gens vorhandene Deletion beseitigt ist und das ein Genprodukt kodiert, welches bei einer Temperatur von > 25° C normale Stabilität aufweist und damit bei einer für *B. thuringiensis* Zellen bevorzugten Kultivierungstemperatur von 30° C bis 33° C in den üblichen Ausbeuten erhältlich ist.

2. Modifiziertes CryIA(b)-Gen gemäss Anspruch 1, dadurch gekennzeichnet, dass besagte Deletion durch Austausch eines entsprechenden Fragments aus dem Wildtyp CyrIA(b)-Gen, welches diese Deletion aufweist, mit einem vollständig oder teilweise überlappenden Fragment aus einem CryIA(a)- oder einem CryIA(c)-Gen ohne Deletion oder einem diesen CryIA-Fragmenten homologen DNA-Fragment, beseitigt ist.

3. Modifiziertes CryIA(b)-Gen gemäss Anspruch 2, dadurch gekennzeichnet, dass die im C-Terminus des Wildtyp Gens vorhandene Deletion durch Austausch eines entsprechenden Fragments aus dem Wildtyp CyrIA(b)-Gen, welches diese Deletion aufweist, mit einem vollständ oder teilweise überlappenden Fragment aus einem CryIA(c)-Gen ohne Deletion oder einem diesem Fragment homologen DNA-Fragment, beseitigt ist.

4. Modifiziertes CryIA(b)-Gen gemäss Anspruch 3, dadurch gekennzeichnet, dass es sich bei besagtem Fragment aus dem CryIA(c)-Gen um ein KpnI-HindIII Fragment handelt.

5. Modifiziertens CryIA(b)-Gen gemäss Anspruch 4, dadurch gekennzeichnet, dass besagtes KpnI-HindIII Fragment einen Bereich umfasst, der sich von Position 2564 bis 3212 auf dem CryIA(c)-Gen erstreckt.

6. Modifiziertes CryIA(b)-Gen gemäss Anspruch 5, das die in Sequenzprotokoll 4 [SEQ ID NO : 4] wiedergegebene DNA Sequenz aufweist sowie Mutanten und Varianten davon, einschliesslich Teilsequenzen, die noch die noch die charakteristischen Eigenschaften des Ausgangsgens aufweisen.

7. Rekombinantes DNA Molekül enthaltend ein modifiziertes CryIA(b)-Gen gemäss einem der Ansprüche 1 bis 6.

8. Rekombinantes DNA Molekül gemäss Anspruch 7, dadurch gekennzeichnet, dass es sich um ein Vektormolekül handelt.

9. Wirtszelle enthaltend ein rekombinantes DNA Molekül gemäss einem der Ansprüche 7 oder 8.

10. *B. thuringiensis* oder *B. cereus*-Zellen, die mit einem rekombinanten DNA-Molekül transformiert sind, das ein modifiziertes CryIA(b)-Strukturgen enthält, welches ein modifiziertes, temperaturstabiles δ-Endotoxin-Polypeptid kodiert, das auch bei einer Kultivierungstemperatur von > 25° C stabil und damit in den üblichen Ausbeuten erhältlich ist, oder aber ein Polypeptid, das diesem im wesentlichen homolog ist.

11. *B. thuringiensis* oder *B. cereus*-Zellen gemäss Anspruch 11,, dadurch gekennzeichnet, dass besagtes modifiziertes, temperaturstabiles δ-Endotoxin-Polypeptid bei einer Temperatur von 30° C bis 33° C stabil und damit in den üblichen Ausbeuten erhältlich ist.

12. Modifiziertes, temperaturstabiles Protoxin, welches von einem modifizierten CryIA(b) Gen gemäss einem der Ansprüche 1 bis 6 kodiert wird und das bei einer Temperatur von > 25° C normale Stabilität aufweist und damit bei einer für *B. thuringiensis* Zellen bevorzugten Kultivierungstemperatur von 30° C bis 33° C in den üblichen Ausbeuten erhältlich ist

**13.** Modifiziertes, temperaturstabiles Protoxin gemäss Anspruch 12, das die in Sequenzprotokoll 5 [EQ ID NO: 5] wiedergegebene Aminosäuresequenz aufweist sowie Mutanten und Varianten davon, einschliesslich Teilsequenzen, die noch die gleichen charakteristischen Eigenschaften wie das Ausgangsmaterial aufweisen.

**14.** Verfahren zur Herstellung eines modifizierten CryIA(b)-Gens, das ein Genprodukt kodiert, welches bei einer Temperatur von > 25° C normale Stabilität aufweist und damit bei einer für *B. thuringiensis* Zellen bevorzugten Kultivierungstemperatur von 30° C bis 33° C in den üblichen Ausbeuten erhältlich ist, dadurch gekennzeichnet, dass man die im C-Terminus des CryIA(b)-Gens vorhandene Deletion durch Austausch eines entsprechenden Fragments aus dem CyrIA(b)-Gen, welches diese Deletion aufweist, mit einem vollständig oder teilweise überlappenden Fragment aus einem CryIA(a)- oder einem CryIA(c)-Gen ohne Deletion oder einem diesen CryIA-Fragmenten homologen DNA-Fragment, beseitigt.

**15.** Verfahren zur Herstellung eines modifizierten CryIA(b)-Gens gemäss Anspruch 14, dadurch gekennzeichnet, dass es sich bei besagtem überlappenden Fragment um ein Fragment aus einem CryIA(c)-Gen handelt oder um ein diesem Fragment homologes DNA-Fragment.

**16.** Verfahren zur Herstellung eines modifizierten CryIA(b)-Gens gemäss Anspruch 15, dadurch gekennzeichnet, dass es sich bei besagtem Fragment um ein KpnI-HindIII Fragment handelt.

**17.** Verfahren zur Herstellung eines modifizierten CryIA(b)-Gens gemäss Anspruch 14, dadurch gekennzeichnet, dass man die folgenden Fragmente in einer Ligasereaktion miteinander verknüpft :
(a) das nach Restriktionsverdauung erhältliche, grosse PstI-KpnI Fragment aus dem Plasmid pXI93, das im wesentlichen den N-terminalen Teil des CryIA(b)-Gens umfasst, nicht jedoch die zu eliminierende Deletion ;
(b) ein 648 Bp umfassendes KpnI-HindIII Fragment aus dem CryIA(c)-Gen, welches eine DNA-Region innerhalb des C-Terminus abdeckt, die der korrespondierenden Region auf dem CryIA(b)-Gen weitgehend homolog ist, mit Ausnahme der dort befindlichen Deletion ;
(c) ein 1460 Bp umfassendes HindIII-PstI Fragment aus dem Plasmid pXI36, welches einen Grossteil des C-Terminus des CryIA(b)-Gens enthält, nicht jedoch die Region mit der Deletion.

**18.** Verfahren zur Herstellung eines modifizierten, temperaturstabilen Protoxins, dadurch gekennzeichnet, dass man
(a) *B. thuringiensis* und/oder *B. cereus* Zellen mit einem rekombinanten DNA Molekül gemäss einem der Ansprüche 7 oder 8 transformiert ;
(b) die transformierten *Bacillus*-Zellen in einem geeigneten Medium kultiviert ; und
(c) nach Sporulation und Lyse der *Bacillus*-Zellen die im Kultivierungsmedium befindlichen Protoxin-Kristalle isoliert.

**19.** Verfahren zur Bekämpfung von Insekten, dadurch gekennzeichnet, dass man Insekten oder deren Lebensraum mit
a) *B. thuringiensis* oder *B. cereus*-Zellen oder mit einem Gemisch von beiden behandelt, die mit einem rekombinanten DNA-Molekül transformiert sind, das ein CryIA(b)-Strukturgen enthält, welches ein modifiziertes, temperaturstabiles δ-Endotoxin-Polypeptid kodiert, das auch bei einer Kultivierungstemperatur von > 25° C stabil und damit bei einer für die Kultivierung der *B. thuringiensis* Zellen bevorzugten Temperatur von 30° C bis 33° C in den üblichen Ausbeuten erhältlich ist, oder aber ein Polypeptid, das diesem im wesentlichen homolog ist ; oder aber
b) mit zellfreien Kristallkörperpräparaten, die ein modifiziertes, temperaturstabiles Protoxin enthalten, das von besagten transformierten *Bacillus*-Zellen produziert wird.

**20.** Insektizide Mittel, die neben den üblicherweise verwendeten Trägermitteln, Verteilungsmitteln oder Träger- und Verteilungsmitteln als aktive Komponente
a) *B. thuringiensis* oder *B. cereus*-Zellen oder ein Gemisch von beiden enthalten, die mit einem rekombinanten DNA-Molekül transformiert sind, das ein CryIA(b)-Saukturgen enthält, welches für ein modifiziertes, temperaturstabiles δ-Endotoxin-Polypeptid kodiert, das auch bei einer Kultivierungstemperatur von > 25° C stabil und damit bei einer für die Kultivierung der *B. thuringiensis* Zellen bevorzugten Temperatur von 30° C bis 33° C in den üblichen Ausbeuten erhältlich ist, oder aber ein Polypeptid, das diesem im wesentlichen homolog ist ; oder aber
b) zellfreie Kristallkörperpräparate, die ein modifiziertes, temperaturstabiles Protoxin enthalten, das von besagten transformierten *Bacillus*-Zellen produziert wird.

**21.** Verfahren zur Herstellung von *B. thuringiensis* und/oder *B. cereus* Zellen, enthaltend ein modifiziertes CryIA(b) Gen gemäss Anspruch 1, dadurch gekennzeichnet, dass man besagte *Bacillus*-Zellen mit einem rekombinanten DNA Molekül gemäss einem der Ansprüche 7 oder 8 transformiert.

**22.** Verfahren zur Herstellung insektizider Mittel dadurch gekennzeichnet dass man eine insektizid wirksame Menge von
a) *B. thuringiensis* oder *B. cereus*-Zellen oder ein Gemisch von beiden enthalten, die mit einem rekombinanten DNA-Molekül transformiert sind, das ein CryIA(b)-Strukturgen enthält, welches für ein modifiziertes, temperaturstabiles δ-Endotoxin-Polypeptid kodiert, das auch bei einer Kultivierungstemperatur von > 25° C stabil und damit bei einer für die Kultivierung der *B. thuringiensis* Zellen bevorzugten Temperatur von 30° C bis 33° C in den üblichen Ausbeuten erhältlich ist, oder aber ein Polypeptid, das diesem im wesentlichen homolog ist ; oder aber
b) zellfreie Kristallkörperpräparate, die ein modifiziertes, temperaturstabiles Protoxin enthalten, das von besagten transformierten *Bacillus*-Zellen produziert wird, mit den üblicherweise verwendeten Trägermitteln, Verteilungsmitteln oder Träger- und Verteilungsmitteln vermischt.

**Patentansprüche Für folgenden Vertraugsstaat : ES.**

**1.** Verfahren zur Herstellung eines modifizierten CryIA(b)-Gens, welches ein temperaturstabiles δ-Endotoxin-Polypeptid kodiert, das auch bei einer Kultivierungstemperatur von > 25° C stabil und damit in den üblichen Ausbeuten erhältlich ist, dadurch gekennzeichnet, dass man die im C-Terminus des CryIA(b)-Gens vorhandene Deletion durch Austausch eines entsprechenden Fragments aus dem CyrIA(b)-Gen, welches diese Deletion aufweist, mit einem vollständig oder teilweise überlappenden Fragment aus einem CryIA(a)- oder einem CryIA(c)-Gen ohne Deletion oder einem diesen CryIA-Fragmenten homologen DNA-Fragment, beseitigt.

**2.** Verfahren zur Herstellung eines modifizierten CryIA(b)-Gens gemäss Anspruch 1, dadurch gekennzeichnet, dass es sich bei besagtem überlappenden Fragment um ein Fragment aus einem CryIA(c)-Gen handelt oder um ein diesem Fragment homologes DNA-Fragment.

**3.** Verfahren gemäss Anspruch 2, dadurch gekennzeichnet, dass es sich bei besagtem Fragment aus dem CryIA(c)-Gen um ein KpnI-HindIII Fragment handelt.

**4.** Verfahren gemäss Anspruch 3, dadurch gekennzeichnet, dass besagtes KpnI-HindIII Fragment einen Bereich umfasst, der sich von Position 2564 bis 3212 auf dem CryIA(c)-Gen erstreckt.

**5.** Verfahren gemäss Anspruch 1, dadurch gekennzeichnet, dass besagtes modifiziertes CryIA(b)-Gen die im Sequenzprotokoll 4 [SEQ ID NO : 4] wiedergegebene DNA Sequenz aufweist.

**6.** Verfahren zur Herstellung eines modifizierten CryIA(b)-Gens gemäss Anspruch 1, dadurch gekennzeichnet, dass man die folgenden Fragmente in einer Ligasereaktion miteinander verknüpft :
(a) das nach Restriktionsverdauung erhältliche, grosse PstI-KpnI Fragment aus dem Plasmid pXI93, das im wesentlichen den N-terminalen Teil des CryIA(b)-Gens umfasst, nicht jedoch die zu eliminierende Deletion ;
(b) ein 648 Bp umfassendes KpnI-HindIII Fragment aus dem CryIA(c)-Gen, welches eine DNA-Region innerhalb des C-Terminus abdeckt, die der korrespondierenden Region auf dem CryIA(b)-Gen weitgehend homolog ist, mit Ausnahme der dort befindlichen Deletion ;
(c) ein 1460 Bp umfassendes HindIII-PstI Fragment aus dem Plasmid pXI36, welches einen Grossteil des C-Terminus des CryIA(b)-Gens enthält, nicht jedoch die Region mit der Deletion.

**7.** Verfahren zur Herstellung eines modifizierten, temperaturstabilen Protoxins, das auch bei einer Kultivierungstemperatur von > 25° C stabil und damit in den üblichen Ausbeuten erhältlich ist, dadurch gekennzeichnet, dass man
(a) *B. thuringiensis* und/oder *B. cereus* Zellen mit einem rekombinanten DNA Molekül enthaltend ein modifiziertes CryIA(b)-Gen gemäss gemäss einem der Ansprüche 1 bis 3 transformiert ;
(b) die transformierten *Bacillus*-Zellen in einem geeigneten Medium kultiviert ; und
(c) nach Sporulation und Lyse der *Bacillus*-Zellen die im Kultivierungsmedium befindlichen Protoxin-Kristalle isoliert.

8. Verfahren gemäss Anspruch 7, dadurch gekennzeichnet, dass besagtes modifiziertes, temperaturstabiles Protoxin die in Sequenzprotokoll 5 [SEQ ID NO : 5] wiedergegebene Aminosäuresequenz aufweist.

9. Verfahren zur Bekämpfung von Insekten, dadurch gekennzeichnet, dass man Insekten oder deren Lebensraum mit
a) *B. thuringiensis* oder *B. cereus*-Zellen oder mit einem Gemisch von beiden behandelt, die mit einem rekombinanten DNA-Molekül transformiert sind, das ein CryIA(b)-Strukturgen enthält, welches ein modifiziertes, temperaturstabiles δ-Endotoxin-Polypeptid kodiert, das auch bei einer Kultivierungstemperatur von > 25° C stabil und damit bei einer für die Kultivierung der *B. thuringiensis* Zellen bevorzugten Temperatur von 30° C bis 33° C in den üblichen Ausbeuten erhältlich ist, oder aber ein Polypeptid, das diesem im wesentlichen homolog ist ; oder aber
b) mit zellfreien Kristallkörperpräparaten, die ein modifiziertes, temperaturstabiles Protoxin enthalten, das von besagten transformierten *Bacillus*-Zellen produziert wird.

10. Verfahren zur Herstellung insektizider Mittel dadurch gekennzeichnet dass man eine insektizid wirksame Menge von
a) *B. thuringiensis* oder *B. cereus*-Zellen oder ein Gemisch von beiden enthalten, die mit einem rekombinanten DNA-Molekül transformiert sind, das ein CryIA(b)-Strukturgen enthält, welches für ein modifiziertes, temperaturstabiles δ-Endotoxin-Polypeptid kodiert, das auch bei einer Kultivierungstemperatur von > 25° C stabil und damit bei einer für die Kultivierung der *B. thuringiensis* Zellen bevorzugten Temperatur von 30° C bis 33° C in den üblichen Ausbeuten erhältlich ist, oder aber ein Polypeptid, das diesem im wesentlichen homolog ist ; oder aber
b) zellfreie Kristallkörperpräparate, die ein modifiziertes, temperaturstabiles Protoxin enthalten, das von besagten transformierten *Bacillus*-Zellen produziert wird, mit den üblicherweise verwendeten Trägermitteln, Verteilungsmitteln oder Träger- und Verteilungsmitteln vermischt.

11. Verfahren zur Herstellung von *B. thuringiensis* und/oder *B. cereus* Zellen, enthaltend ein modifiziertes CryIA(b) Gen gemäss Anspruch 1, dadurch gekennzeichnet, dass man besagte *Bacillus*-Zellen mit einem rekombinanten DNA Molekül transformiert, welches ein CryIA(b)-Strukturgen enthält, das ein modifiziertes, temperaturstabiles δ-Endotoxin-Polypeptid kodiert, das auch bei einer Kultivierungstemperatur von > 25° C stabil und damit in den üblichen Ausbeuten erhältlich ist, oder aber ein Polypeptid, das diesem im wesentlichen homolog ist.

12. Verfahren gemäss Anspruch 11, dadurch gekennzeichnet, dass besagtes rekombinantes DNA Molekül ein modifiziertes CryIA(b) Gen enthält, welches ein modifiziertes, temperaturstabiles δ-Endotoxin-Polypeptid kodiert, das bei einer für die Kultivierung der *B. thuringiensis* Zellen bevorzugten Temperatur von 30° C bis 33° C stabil und damit in den üblichen Ausbeuten erhältlich ist.

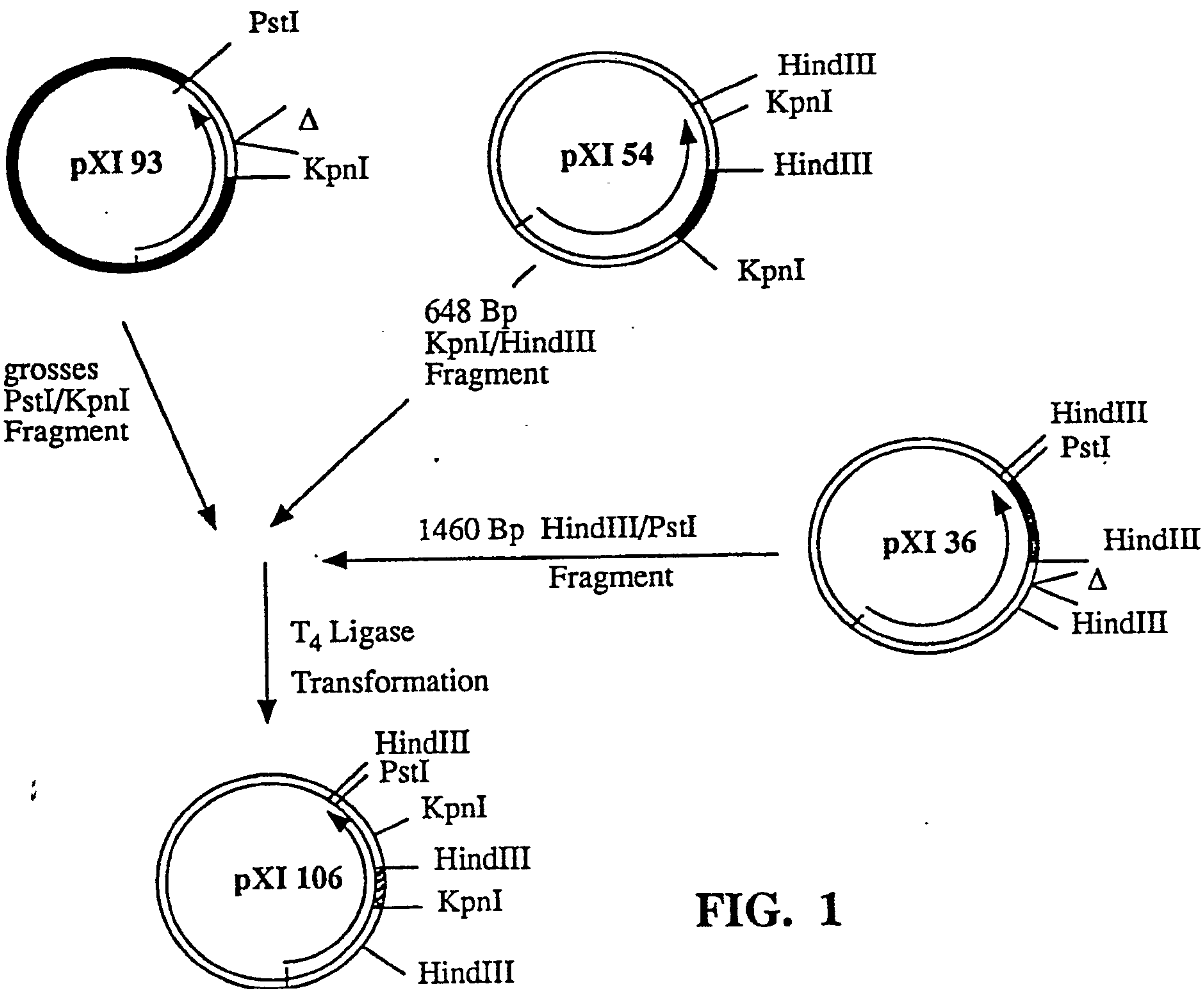
PstI
Δ
KpnI
pXI 93
HindIII
KpnI
pXI 54
HindIII
KpnI
648 Bp
KpnI/HindIII
Fragment
grosses
PstI/KpnI
Fragment
HindIII
PstI
1460 Bp HindIII/PstI
Fragment
pXI 36
HindIII
Δ
HindIII
$T_4$ Ligase
Transformation
HindIII
PstI
KpnI
pXI 106
HindIII
KpnI
HindIII

FIG. 1

Europäisches Patentamt

# EUROPÄISCHER RECHERCHENBERICHT

Nummer der Anmeldung

EP 91 81 0050
Seite 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | EP-A-228838 (MYCOGEN CORPORATION)<br>* das ganze Dokument *<br>--- | 1-3, 7-15, 19-22 | C12N15/32<br>C12N15/62<br>A01N63/00 |
| D,A | GENE.<br>vol. 48, 1986, AMSTERDAM NL<br>Seiten 109 - 118; M.Geiser et al.:<br>"The hypervariable region in the genes coding for entomopathogenic crystal proteins of Bacillus thuringiensis:nucleotide sequence of the kurhdl gene of subsp. kurstaki HD1"<br>* Seite 114, Zeilen 19 - 21; Figuren 4a, 4b *<br>--- | 1-6 | |
| D,A | GENE.<br>vol. 36, 1985, AMSTERDAM NL<br>Seiten 289 - 300; M.J.Adang et al.:<br>"Characterized full-length and truncated plasmid clones of the crystal protein of Bacillus thuringiensis subsp. kurstaki HD-73 and their toxicity to Manduca sexta"<br>* Zusammenfassung; Figur 4 *<br>--- | 1-6, 19, 20 | **RECHERCHIERTE SACHGEBIETE (Int. Cl.5 )** |
| A | PATENT ABSTRACTS OF JAPAN<br>vol. 12, no. 391 (C-537)(3238) 18 Oktober 1988,<br>& JP-A-63 137684 (SUMITOMO CHEM CO LTD) 09 Juni 1988,<br>* das ganze Dokument *<br>--- | 1-22 | C12N<br>C07K |
| A | MOLECULAR MICROBIOLOGY<br>vol. 3, no. 11, 1989, NEW YORK,US<br>Seiten 1533 - 1543; H.Arvidson et al.:<br>"Specificity of Bacillus thuringiensis for lepidopteran larvae: factors involved in vivo and in the structure of a purified protoxin"<br>* Zusammenfassung *<br>* Seite 1528, rechte Spalte, Zeilen 4 - 46 *<br>---<br>-/-- | 1, 9, 10, 19, 20 | |

Der vorliegende Recherchenbericht wurde für alle Patentansprüche erstellt

| Recherchenort | Abschlußdatum der Recherche | Prüfer |
|---|---|---|
| BERLIN | 24 APRIL 1991 | GURDJIAN D. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 91 81 0050
Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | GENE. vol. 43, 1986, AMSTERDAM NL Seiten 29 - 40; J.W.Kronstad et al.: "Three classes of homologous Bacillus thuringiensis crystal-protein genes" * Zusammenfassung; Figur 4 * | 1 | |

RECHERCHIERTE SACHGEBIETE (Int. Cl.5)

Der vorliegende Recherchenbericht wurde für alle Patentansprüche erstellt

| Recherchenort | Abschlußdatum der Recherche | Prüfer |
|---|---|---|
| BERLIN | 24 APRIL 1991 | GURDJIAN D. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)